(19) Europäisches Patentamt / European Patent Office / Office européen des brevets

(11) EP 4 065 080 B1

(12) **EUROPÄISCHE PATENTSCHRIFT**

(45) Veröffentlichungstag und Bekanntmachung des Hinweises auf die Patenterteilung:
**19.06.2024 Patentblatt 2024/25**

(21) Anmeldenummer: **20772044.2**

(22) Anmeldetag: **14.09.2020**

(51) Internationale Patentklassifikation (IPC):
***A61K 8/898*** (2006.01) ***A61Q 5/02*** (2006.01)
***A61Q 5/06*** (2006.01) ***A61K 8/46*** (2006.01)

(52) Gemeinsame Patentklassifikation (CPC):
**A61Q 5/065; A61K 8/463; A61K 8/898; A61Q 5/02;**
A61K 2800/884

(86) Internationale Anmeldenummer:
**PCT/EP2020/075595**

(87) Internationale Veröffentlichungsnummer:
**WO 2021/104701 (03.06.2021 Gazette 2021/22)**

(54) **VERBESSERUNG DER LEISTUNG VON AUF PIGMENTEN BASIERENDEN FÄRBEMITTELN DURCH ANWENDUNG EINES VORBEHANDLUNGSMITTELS**

IMPROVING THE PERFORMANCE OF PIGMENT-BASED DYES BY USING A PRETREAMENT AGENT

AMÉLIORATION DE LA PERFORMANCE DE COLORANTS À BASE DE PIGMENT À L’AIDE D’UN AGENT DE PRÉTRAITEMENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.11.2019 DE 102019218232**

(43) Veröffentlichungstag der Anmeldung:
**05.10.2022 Patentblatt 2022/40**

(73) Patentinhaber: **Henkel AG & Co. KGaA**
**40589 Düsseldorf (DE)**

(72) Erfinder:
- **KRUCK, Constanze**
  **41515 Grevenbroich (DE)**
- **HILBIG, Sandra**
  **44894 Bochum (DE)**
- **MOCH, Melanie**
  **41542 Dormagen (DE)**
- **DICKHOF, Susanne**
  **41748 Viersen (DE)**
- **KESSLER-BECKER, Daniela**
  **51371 Leverkusen (DE)**

(56) Entgegenhaltungen:
**WO-A2-2020/008073 GB-A- 2 291 366**
**US-A1- 2016 235 655**

- **DATABASE GNPD [Online] MINTEL; 9. Januar 2013 (2013-01-09), anonymous: "Shampoo for Fine/Normal Hair", XP055753010, Database accession no. 1964692**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Processed by Luminess, 75001 PARIS (FR)

**Beschreibung**

Gegenstand der vorliegenden Anmeldung ist ein Verfahren zum Färben von keratinischem Material, insbesondere menschlichen Haaren, welches die Anwendung von mindestens zwei verschiedenen Mitteln (V) und (F) umfasst. Das Mittel (V) stellt ein Vorbehandlungsmittel dar, welches in einem wasserhaltigen Träger mindestens ein anionisches Tensid enthält. Das Färbemittel (F) enthält mindestens ein aminofunktionalisiertes Silikonpolymer (F-1) und mindestens ein Pigment (F-2).

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren. Ein mögliches, alternatives Färbesystem, das in letzter Zeit zunehmend in den Fokus rückt, beruht auf dem Einsatz von farbigen Pigmenten.

Die Färbung mit Pigmenten bietet verschiedene wesentliche Vorteile. Da die Pigmente sich lediglich von außen an die Keratinmaterialen, insbesondere an die Haarfasern, anlagern, ist die mit dem Färbeprozess verbundene Schädigung ganz besonders gering. Weiterhin lassen sich nicht mehr erwünschte Färbungen schnell und einfach rückstandslos entfernen und bieten dem Anwender auf diese Weise die Möglichkeit, unmittelbar und ohne großen Aufwand zu seiner Ursprungshaarfarbe zurückkehren zu können. Insbesondere für die Konsumenten, die ihre Haare nicht regelmäßig nachfärben möchten, ist dieser Färbeprozess daher besonders attraktiv.

US 2016/235655 A1 beschreibt ein Verfahren zur Ausbildung eines Films auf Keratinfasern, bei dem eine Zusammensetzung enthaltend ein bestimmtes Aminosilikon, ein MQ-Harz, ein Verdicker- System, ein Pigment und ein Lösungsmittel auf die Keratinfasern appliziert wird. Im Beispielteil werden entsprechende Zusammensetzungen auf Haarsträhnen angewendet ([0236]) und danach mit dem Shampoo "Wella Professionals Brilliance" gewaschen ([00243])

In aktuellen Arbeiten wurde das Problem der geringen Haltbarkeit dieses Färbesystems adressiert.

In diesem Zusammenhang konnte gefunden werden, dass die Waschechtheit der mit Pigmenten erhaltenen Farbresultate durch Kombination der Pigmente mit bestimmten aminofunktionalisierten Silikonpolymeren stark verbessert werden konnte. Darüber hinaus konnte durch die Wahl besonders gut geeigneter Pigmente und Pigmentkonzentrationen auf dunklem Haar ein helleres Farbergebnis erzielt werden, so dass mit diesem Färbesystem sogar eine Aufhellung möglich wurde, die bis dato ausschließlich mit oxidativen Haarbehandlungsmitteln (Bleich- bzw. Blondiermitteln) möglich war.

Neben diesen vielen Vorteilen besitzt das auf Pigmenten basierende Färbesystem jedoch immer noch einige Nachteile. Da sowohl die Pigmente als auch die Aminosilikone, welche die Pigmente immobilisieren, sich auf der Oberfläche der Haarfaser ablagern, ist die Erzielung eines einheitlichen Farbegebnisses in hohem Maße von der Oberflächenstruktur der zu färbenden Haare abhängig. Die in diesem Zusammenhang durchgeführten Arbeiten haben gezeigt, dass auch Substanzen, die sich zum Zeitpunkt der Färbung noch auf der Oberfläche der Haare befinden, eine sehr starke Auswirkung auf die Qualität eines nachfolgenden Färbeergebnisses haben können. So wurden beispeilsweise bei Personen, die ihre Haare zuvor mit einem Conditioner oder Stylingmittel behandelt hatten, Färbungen mit geringer Intensität und extrem ungleichmäßigem Farbausfall erhalten. Ohne auf diese Theorie festgelegt zu sein, wird vermutet, dass verschiedene in Pflege- und/oder Stylingmitteln enthaltene Inhaltsstoffe sich an bestimmte Stellen des Haares anlagern können und dort in einem späteren Färbeprozess die Ausbildung von gleichmäßigen Farbfilmen verhindern.

Der Anwender wünscht sich intensive Färbungen mit guter Gleichmäßigkeit und hoher Reproduzierbarkeit. Dementsprechend ist ein Färbesystem, dessen Farbergebnis von der Art und Menge eines anderen möglicherweise zuvor angewendeten kosmetischen Produktes abhängt, in den Augen des Anwenders in hohem Maße unattraktiv.

Es war die Aufgabe der vorliegenden Anmeldung, ein auf Pigmenten basierendes Färbeverfahren aufzufinden, mit dem sich intensive Färbungen mit guter Reproduzierbarkeit erzielen lassen. Hierbei sollten die mit diesem Verfahren gefärbten Keratinmatieralien bzw. insbesondere Haare unabhängig vom Haartyp und von anderen zuvor angewendeten kosmetischen Produkten Färbungen in genau der Nuance und Intensität liefern, die der Anwender nach Lekture der entsprechenden Verpackungsinformation bzw. Gebrauchsanweisung auch erwaret. Die Färbungen sollten sich auch bei vorheriger Anwendung eines Pflege- oder Styling mittels durch eine hohe Gleichmäßigkeit und Egalisierung auszeichnen.

Überraschenderweise hat sich nun herausgestellt, dass die vorgenannte Aufgabe hervorragend gelöst werden kann, wenn keratinische Materialien, insbesondere Haare, mit einem zweistufigen Verfahren gefärbt werden, bei welchem mindestens zwei Mittel (V) und (F) auf die keratinischen Materialien (insbesondere Keratinfasern wie Haare) appliziert werden. Hierbei handelt es sich bei dem Mittel (V) um ein Vorbehandlungsmittel, das in einem wässrigen oder wasserhaltigen Träger mindestens ein anionisches Tensid (V-1) enthält. Das Vorbhandlungsmittel (V) wird vor der Anwendung des eigentlichen Färbemittels (F) auf die Keratinmaterialien aufgetragen, einwirken gelassen und wieder ausgespült. Im Anschluss an die Applikation des Vorbehandlungsmittels (V) wird die Färbung durch das Auftragen des eigentlichen Färbemittels (F) auf das Keratinmaterial vorgenommen. Das Färbemittel (F) ist gekennzeichnet durch seinen Gehalt an mindestens einem aminofunktionalisiertes Silikonpolymer (F-1) und mindestens einem Pigment (F-2).

Ein erster Gegenstand der vorliegenden Erfindung ist ein Verfahren zum Färben von keratinischen Fasern, insbesondere menschlichen Haaren, umfassend die folgenden Schritte in der angegebenen Reihenfolge:

(1) Applizieren eines Vorbehandlungsmittels (V) auf die keratinischen Fasern, wobei das Vorbehandlungsmittel in einem wasserhaltigen Träger
(V-1) mindestens ein anionisches Tensid enthält,
(2) Einwirken des in Schritt (1) applizierten Vorbehandlungsmittels auf die keratinischen Fasern,
(3) Ausspülen des Vorbehandlungsmittels mit Wasser,
(4) Applizieren eines Färbemittels (F) auf die keratinischen Fasern, wobei das Färbemittel (F-1) mindestens ein aminofunktionalisiertes Silikonpolymer und (F-2) mindestens ein Pigment enthält,
(5) Einwirken des in Schritt (4) applizierten Färbemittels auf die keratinischen Fasern, und
(6) Ausspülen des Färbemittels mit Wasser, dadurch gekennzeichnet, dass zwischen den Schritten (3) und (4) ein Zeitraum von maximal 3 Stunden liegt.

Bei den zu dieser Erfindung führenden Arbeiten hat sich gezeigt, dass besonders intensive und gleichmäßige Farbergebnisse auf Haaren erhalten werden können, wenn die Haare durch suksessive Anwendung der beiden Mittel (V) und (F) gefärbt wurden. Überraschenderweise wurden die Intensität und die Gleichmäßigkeit des Farbergebnisses auch bei zuvor erfolgter Anwendung eines Conditioners oder Styling mittels nicht beeinträchtigt.

keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

Mittel zur Färbung

Der Begriff "Mittel zur Färbung" wird im Rahmen dieser Erfindung für eine durch Einsatz von Pigmenten hervorgerufene Farbgebung des Keratinmaterials, insbesondere des Haares, verwendet. Bei dieser Färbung lagern sich die Pigmente als farbgebende Verbindungen in einem besonders homogenen, gleichmäßigen und glatten Film an der Oberfläche des Keratinmaterials ab.

Vorbehandlungsmittel (V)

Vor Anwendung des Färbemittels (F) wird im Erfindungsgemäßen Verfahren das Vorbehandlungsmittel (V) auf die keratinischen Materialien, insbesondere auf die Keratinfasern, appliziert.

Das Vorbehandlungsmittel (V) ist dadurch gekennzeichnet, dass es in einem wässrigen oder wasserhaltigen

Träger mindestens ein anionisches Tensid (V-1) enthält.

Wassergehalt im Vorbehandlungsmittel

Das Vorbehandlungsmittel (V) enthält das oder die anionischen Tenside (V-1) in einem wässrigen oder wasserhaltigen Träger. Bevorzugt besitzt das Vorbehandlungsmittel (V) einen hohen Wasseranteil. Es hat sich herausgestellt, dass besonders die Vorbehandlungsmittel (V) zum Einsatz im erfindungsgemäßen Verfahren gut geeignet sind, die - bezogen auf das Gesamtgewicht des Vorbehandlungsmittel (V) - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthalten.

In einer Ausführungsform ist ein erfindungsgemäßes Verfahren dadurch gekennzeichnet, dass das Vorbehandlungsmittel (V) - bezogen auf das Gesamtgewicht des Vorbehandlungsmittels (V) - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthält.

Aninionische Tenside (V-1) im Vorbehandlungsmittel

Als wesentlichen Inhaltssstoff enthält das erfindungsgemäße Vorbehandlungsmittel mindestens ein anionisches Tensid (V-1).

Unter dem Begriff Tenside (T) werden grenzflächenaktive Substanzen, die an Ober- und Grenzflächen Adsorptionsschichten bilden oder in Volumenphasen zu Mizell-Kolloiden oder lyotropen Mesophasen aggregieren können, verstanden. Man unterscheidet anionische Tenside bestehend aus einem hydrophoben Rest und einer negativ geladenen hydrophilen Kopfgruppe, amphotere bzw. zwitterionische Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Bestimmte anionische Tenside (V-1) haben sich als besonders gut geeignet herausgestellt, um bei nachfolgender Anwendung eines erfindungsgemäßen Färbemittels (F) ein intensives, gleichmäßiges und reproduzierbares Farbergebnis zu erzielen.

Zu den geeigneten anionischen Tensiden (V-1), die in den erfindungsgemäßen Mitteln eingesetzt werden können, zählen:

- lineare und verzweigte Fettsäuren mit 8 bis 30 C-Atomen (Seifen),
- Ethercarbonsäuren der Formel $R-O-(CH_2-CH_2O)_x-CH_2-COOH$, in der R eine lineare oder verzweigte, gesättigte oder ungesättigte Alkylgruppe mit 8 bis 30 C-Atomen und x = 0 oder 1 bis 16 ist,
- Acylsarcoside mit 8 bis 24 C-Atomen in der Acylgruppe,
- Acyltauride mit 8 bis 24 C-Atomen in der Acylgruppe,
- Acylisethionate mit 8 bis 24 C-Atomen in der Acylgruppe,
- Sulfobernsteinsäuremono- und/oder -dialkylester mit 8 bis 24 C-Atomen in der Alkylgruppe und Sulfobernsteinsäuremono-alkylpolyoxyethylester mit 8 bis 24 C-Atomen in der Alkylgruppe und 1 bis 6 Oxyethylgruppen,
- Alpha-Olefinsulfonate mit 8 bis 24 C-Atomen,
- Alkylsulfat- und/oder Alkylpolyglykolethersulfatsalze der Formel $R-(OCH_2-CH_2)_x-OSO_3^- X^+$, in der R eine bevorzugt lineare oder verzweigte, gesättigte oder ungesättigte Alkylgruppe mit 8 bis 30 C-Atomen, x = 0 oder 1 bis 12 und X ein Alkali- oder Ammoniumion ist,
- Sulfonate ungesättigter Fettsäuren mit 8 bis 24 C-Atomen und 1 bis 6 Doppelbindungen,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen,
- Alkyl- und/oder Alkenyletherphosphate der Formel,

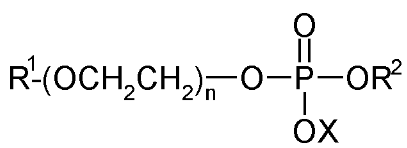

in der $R^1$ bevorzugt für einen aliphatischen Kohlenwasserstoffrest mit 8 bis 30 Kohlenstoffatomen, $R^2$ für Wasserstoff, einen Rest $(CH_2CH_2O)_nR^1$ oder X, n für Zahlen von 0 bis 10 und X für Wasserstoff, ein Alkali- oder Erdalkalimetall oder $NR^3R^4R^5R^6$, mit $R^3$ bis $R^6$ unabhängig voneinander stehend für einen $C_1$ bis $C_4$ -Kohlenwasserstoffrest, steht.

Ganz besonders gute Ergebnisse wurden erhalten, wenn im Vorbehandlungsmittel (V) mindestens ein mindestens ein anionisches Tensid (V-1) der Formel (T-1) eingesetzt wurde,

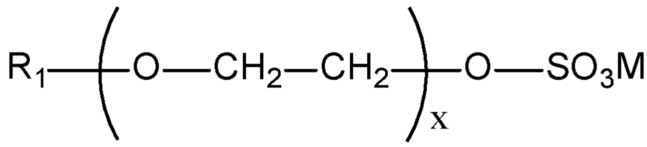

(T-1),

wobei

- $R^1$ für eine lineare oder verzweigte, gesättigte oder ungesättigte $C_8$-$C_{30}$-Alkylgruppe steht,
- x für eine ganze Zahl von 0 bis 50 steht, und
- M für ein Wasserstoffatom, für Ammonium ($NH_4$), oder für ein Äquivalent eines ein oder mehrwertigen Kations steht.

Im Rahmen einer ganz besonders bevorzugten Ausführungsform ist ein erfindungsgemäßes Verfahren dadurch gekennzeichnet, dass das Vorbehandlungsmittel (V)
(V-1) mindestens ein anionisches Tensid der Formel (T-1) enthält,

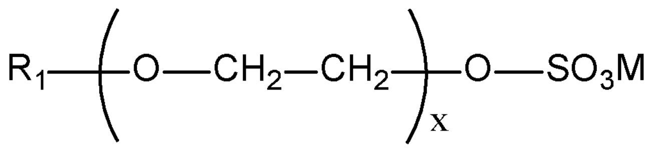

(T-1),

wobei

- $R^1$ für eine lineare oder verzweigte, gesättigte oder ungesättigte $C_8$-$C_{30}$-Alkylgruppe steht,
- x für eine ganze Zahl von 0 bis 50 steht, und
- M für ein Wasserstoffatom, für Ammonium ($NH_4$), oder für ein Äquivalent eines ein oder mehrwertigen Kations steht.

Der Rest R1 stellt den hydrophoben Teil des anionischen Tensids dar und steht für eine lineare oder verzweigte, gesättigte oder ungesättigte $C_8$-$C_{30}$-Alkylgruppe. Wenn der Rest für eine ungesättigte $C_8$-$C_{30}$-Alkylgruppe steht, dann kann die Alkylgruppe einfach oder mehrfach ungesättigt sein.

Bevorzugt steht der Rest R1 für eine lineare, gesättigte oder ungesättigte $C_8$-$C_{30}$-Alkylgruppe- Ganz besonders bevorzugt steht der Rest R1 für eine lineare, gesättigte oder ungesättigte $C_{12}$-$C_{22}$-Alkylgruppe. Explizit ganz besonders bevorzugt steht der Rest R1 für eine R für eine lineare, gesättigte oder ungesättigte $C_{12}$-$C_{18}$-Alkylgruppe.

Beispiele für gesättigte, lineare $C_8$-$C_{30}$-Alkylgruppen sind die Laurylgruppe, die Myristylgruppe, die Cetylgruppe, die Stearylgruppe und die Behenylgruppe.

Die Indexzahl x gibt die Anzahl der im anionischen Tensid enthatlenen Ethylenoxid-Gruppen an. Steht x für die Zahl 0, dann besitzt das anionische Tensid der Formel (T-1) keine EthylenoxidEinheiten, in diesem Fall liegt ein Alkyl-Sulfat bzw. das Salz eines Alkyl-Sulfats vor.

Beispiele für die Salzs von Alkylsulfaten sind Natriumlaurylsulfat und Natriummyristylsulfat.

Bevorzugt steht x für eine ganze Zahl von 0 bis 5, besonders bevorzugt steht x für eine ganze Zahl von 1 bis 5.

Der Rest M steht für ein Wasserstoffatom, für Ammonium ($NH_4$), oder für ein Äquivalent eines ein oder mehrwertigen Kations.

Steht M für ein Wasserstoffatom, dann liegt das anionische Tensid in Form des protonierten (und gegebenenfalls ethoxylierten) Schwefelsäureesters vor. In wässriger Lösung steht die protonierte Form mit der deprotonierten Form im Gleichgewicht, wobei die deprotonierte Form eine anionische Ladung trägt. Aus diesem Grund fallen auch die protonierten Verbindungen der Formel (I) in die Gruppe der anionischen Tenside.

Steht M für Ammonium ($NH_4$), oder für ein Äquivalent eines ein oder mehrwertigen Kations, dann liegt das anionsiche Tensid der Formel (I) in Form seines Salzes vor. Das Vorhandensein der entsprechenden Äquivalent eines ein- oder mehrwertigen Kations gewährleistet hierbei die Elektroneutralität des Anion-Tensid. Bevorzugt steht M für ein einwertiges Kation, insbesondere für ein Natrium- oder Kalium-Kation.

Im Rahmen einer ganz besonders bevorzugten Ausführungsform ist ein erfindungsgemäßes Verfahren dadurch gekennzeichnet, dass das Vorbehandlungsmittel (V) mindestens ein anionisches Tensid (V-1) der Formel (T-1) enthält,

wobei

- R1 für eine lineare, gesättigte oder ungesättigte $C_{12}$-$C_{18}$-Alkylgruppe steht,
- x für eine ganze Zahl von 0 bis 5, bevorzugt für eine ganze Zahl von 1 bis 5, steht,
- M für ein Wasserstoffatom oder für ein Alkalimetallkation, bevorzugt für ein Natriumkation oder für ein Kaliumkation, steht.

Ein ganz besonders bevorzugtes anionisches Tensid der Formel (I) kann beispielsweise unter dem Handelsnamen Texapon NSO BZ (BZ = preserved with benzoic acid) und der INCI Bezeichnung Sodium Laureth Sulfate von der Firma BASF kommerziell bezogen werden. Dieses Sodium Laureth Sulfate besitzt die CAS-Nummer 68891-38-3.

Zur Gewährleistung besonders guter Farbresultate im erfindungsgemäßen Verfahren enthält das Vorbehandlungsmittel das bzw. die anionischen Tenside (V-1) bevorzugt in bestimmten Mengenbereichen. Besonders intensive und gleichmäßige Färbungen konnten erhalten werden, wenn das Vorbehandlungsmittel (V) - bezogen auf das Gesamtgewicht des Vorbehandlungsmittels - ein oder mehrere anionische Tenside (V-1) in einer Gesamtmenge von 2,0 bis 18,0 Gew.-%, bevorzugt von 4,0 bis 16,0 Gew.-%, weiter bevorzugt von 6,0 bis 14,0 Gew.-% und ganz besonders bevorzugt von 8,0 bis 12,0 Gew.-% enthielt

Im Rahmen einer ganz besonders bevorzugten Ausführungsform ist ein erfindungsgemäßes Verfahren dadurch gekennzeichnet, dass das Vorbehandlungsmittel (V) - bezogen auf das Gesamtgewicht des Vorbehandlungsmittels - ein oder mehrere anionische Tenside (V-1) in einer Gesamtmenge von 2,0 bis 18,0 Gew.-%, bevorzugt von 4,0 bis 16,0 Gew.-%, weiter bevorzugt von 6,0 bis 14,0 Gew.-% und ganz besonders bevorzugt von 8,0 bis 12,0 Gew.-% enthält.

<u>zwitterionische und/oder amphotere Tenside im Vorbehandlungsmittel</u>

Die mit dem erfindungsgemäßen Verfahren erzielbaren Farbeffekte ließen sich im Hinblick auf Intensität, Gleichmäßigkeit und Reproduzierbarkeit noch weiter verbessern, wenn im Vorbehandlungsmittel (V) nicht nur mindestens ein anionisches Tensid (V-1), sondern darüber hinaus weiterhin mindestens ein zwitterionisches und/oder amphoteres Tensid eingesetzt wurde.

Im Rahmen einer ganz besonders bevorzugten Ausführungsform ist ein erfindungsgemäßes Verfahren dadurch gekennzeichnet, dass das Vorbehandlungsmittel (V)
(V-2) mindestens ein zwitterionisches und/oder amphoteres Tensid enthält.

Ganz besonders gut geeignete zwitterionische Tenside (V-2) können ausgewählt sein aus Verbindungen der folgenden Formeln (T-2), (T-3), (T-4) und/oder (T-5),

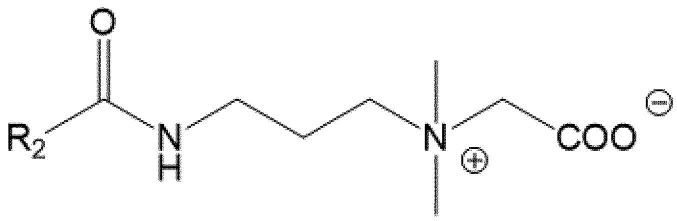

(T-2)

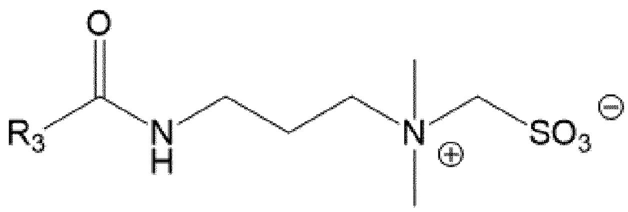

(T-3)

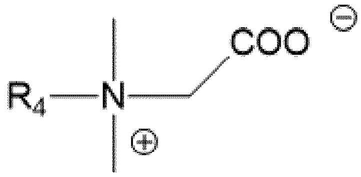

(T-4)

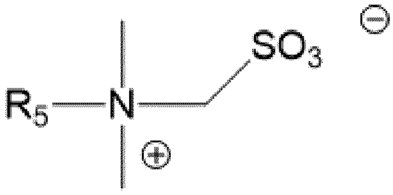

(T-5)

wobei

R2, R3, R4 und R5 jeweils unabhängig voneinander den hydrophoben Rest des Tensids darstellen. R2, R3, R4 und R5 stehen unabhängig voneinander für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe.

Die zwitterionischen Tenside der Formel (T-2), (T-3), (T-4) und/oder (T-5) besitzen jeweils eine kationische Ladung in Form eines quartären Stickstoffatoms, welches neben zwei Methylgruppen den jeweiligen Rest R und weiterhin den Rest umfassend die Säurefunktion trägt. Diese kationische Ladung wird durch die Säurefunktion, bei der es sich um eine deprotonierte Carbonsäure oder Sulfonsäure handeln kann, neutralisiert.

Im Rahmen einer ganz besonders bevorzugten Ausführungsform ist ein erfindungsgemäßes Verfahren dadurch gekennzeichnet, dass das Vorbehandlungsmittel (V)

(V-2) mindestens ein zwitterionisches Tensid enthält, das ausgewählt ist aus den Tensiden der Formel (T-2), (T-3), (T-4) und/oder (T-5),

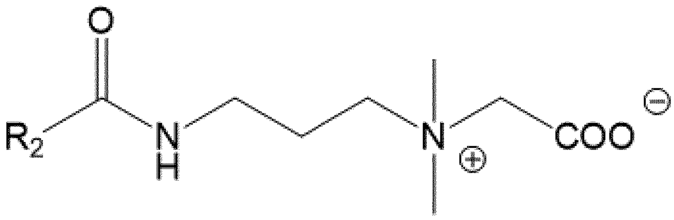

(T-2)

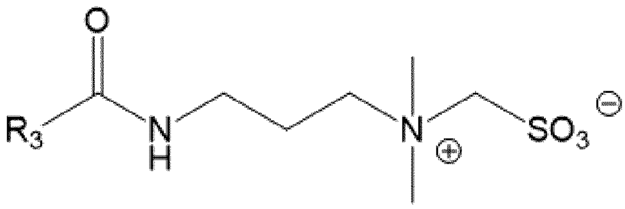

(T-3)

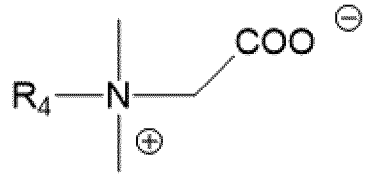

(T-4)

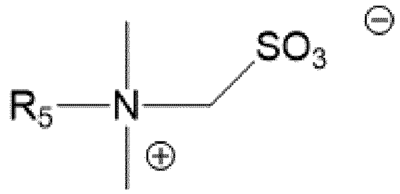

(T-5)

wobei

R2, R3, R4, R5 unabhängig voneinander für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe, stehen.

Der Rest R2 steht für eine lineare oder verzweigte, gesättigte oder ungesättigte $C_8$-$C_{30}$-Alkylgruppe, bevorzugt für eine lineare, gesättigte oder ungesättigte $C_{11}$-$C_{21}$-Alkylgruppe, ganz besonders bevorzugt für eine lineare, gesättigte oder ungesättigte $C_{11}$-$C_{17}$-Alkylgruppe

Der Rest R3 steht für eine lineare oder verzweigte, gesättigte oder ungesättigte $C_8$-$C_{30}$-Alkylgruppe, bevorzugt für eine lineare, gesättigte oder ungesättigte $C_{11}$-$C_{21}$-Alkylgruppe, ganz besonders bevorzugt für eine lineare, gesättigte oder ungesättigte $C_{11}$-$C_{17}$-Alkylgruppe

Der Rest R4 steht für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe.

Der Rest R5 steht für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe.

Besonders geeignete zwitterionische Tenside der Formel (T-2) sind Alkylamidoalkylbetaine. Zu den insbesondere geeigneten amphoteren Tensiden zählen die unter der INCI-Bezeichnung bekannten Tenside Cocamidopropylbetain und der INCI Bezeichnung Cocamidopropyl Betaine.

Besonders geeignette zwitterionische Tenside der Formel (T-3) sind beispielsweise $C_{12}$-$C_{14}$-Alkyldimethylbetaine, die unter dem INCI Namen Coco-Betaine in Form des Handelsproduktes Genagen KB von der Firma Global Amines (ehemals Clariant) bezogen werden können. Coco-Betain besitzt die CAS-Nummer 66455-29-6.

Insbesondere die zwitterionischen Tenside der Formel (T-2) und (T-4) haben zur Lösung der erfindungsgemäßen Aufgabenstellung eine besonders gute Eignung gezeigt.

Im Rahmen einer weiteren ganz besonders bevorzugten Ausführungsform ist ein erfindungsgemäßes Verfahren dadurch gekennzeichnet, dass das Vorbehandlungsmittel (V)

(V-2) mindestens ein zwitterionisches Tensid enthält, das ausgewählt ist aus den Tensiden der Formel (T-2) und/oder (T-4).

Um mit dem erfindungsgemäßen Verfahren besonders gute Färberesultate zu erzielen, enthält das Vorbehandlungsmittel die amphoteren bzw. zwitterionischen Tenside (V-2) bevorzugt in bestimmten Mengenbereichen. Besonders intensive und gleichmäßige Färbungen konnten erhalten werden, wenn das Vorbehandlungsmittel (V) - bezogen auf das Gesamtgewicht des Vorbehandlungsmittels - ein oder mehrere zwitterionische Tenside (V-2) in einer Gesamtmenge von 0,5 bis 8,5 Gew.-%, bevorzugt 1,0 bis 7,5 Gew.-%, weiter bevorzugt 1,5 bis 6,5 Gew.-% und ganz besonders bevorzugt 2,0 bis 4,5 Gew.-% enthielt

Im Rahmen einer ganz besonders bevorzugten Ausführungsform ist ein erfindungsgemäßes Verfahren dadurch gekennzeichnet, dass das Vorbehandlungsmittel (V) - bezogen auf das Gesamtgewicht des Vorbehandlungsmittels - ein oder mehrere zwitterionische und/oder amphotere Tenside (V-2) in einer Gesamtmenge von 0,5 bis 8,5 Gew.-%, bevorzugt 1,0 bis 7,5 Gew.-%, weiter bevorzugt 1,5 bis 6,5 Gew.-% und ganz besonders bevorzugt 2,0 bis 4,5 Gew.-% enthält.

Färbemittel (F)

Im Anschluss an die Anwendung des Vorbehandlungsmittel (V) wird im erfindungsgemäßen Verfahren nun das Färbemittel (F) auf das Keratinmaterial appliziert. Das Färbemittel (F) enthält mindestens ein aminofunktionalisiertes Silikonpolymer (F-1) und mindestens ein Pigment (F-2).

aminofunktionalisiertes Silikonpolymer (F-1) im Färbemittel (F)

Als ersten erfindungswesentlichen Inhaltsstoff (F-1) enthält das Färbemittel (F) mindestens ein aminofunktionalisiertes Silikonpolymer. Das aminofunktionalisiertes Silikonpolymer kann alternativ auch als Aminosilikon oder Amodimethicone bezeichnet werden.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikonpolymers nicht mehr als $10^7$ g/mol, bevorzugt nicht mehr als $10^6$ g/mol und besonders bevorzugt nicht mehr als $10^5$ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Unter einem aminofunktionalisierten Silikonpolymer wird ein funktionalisiertes Silikon verstanden, welches mindestens eine Struktureinheit mit einer Aminogruppe trägt. Bevorzugt trägt das aminofunktionalisierte Silikonpolymer mehrere Struktureinheiten mit jeweils mindestens einer Aminogruppe. Unter einer Aminogruppe wird eine primäre Aminogruppe, eine sekundäre Aminogruppe und eine tertiäre Aminogruppe verstanden. Alle diese Aminogruppen können im sauren Milieu protoniert werden und liegen dann in ihrer kationischen Form vor.

Prinzipiell konnten gute Effekte aminofunktionalisierten Silikonpolymeren (F-1) erzielt werden, wenn diese mindestens eine primäre, mindestens eine sekundäre und/oder mindestens eine tertiäre Aminogruppe tragen. Färbungen mit der besten Waschechtheit wurden jedoch beobachtet, wenn ein aminofunktionalisiertes Silikonpolymer (F-1) im Färbemittel (F) eingesetzt wurde, welches mindestens eine sekundäre Aminogruppe enthält.

In einer ganz besonders bevorzugten Ausführungsform ist ein erfindungsgemäßes Verfahren dadurch gekennzeichnet, dass das Färbemittel (F) mindestens ein aminofunktionalisiertes Silikonpolymer (F-1) mit mindestens eine sekundären Aminogruppe enthält.

Die sekundäre Aminogruppe(n) kann bzw. können sich an verschiedenen Positionen des aminofunktionalisierten Silikonpolymers befinden. Ganz besonders gute Effekte wurden gefunden, wenn ein aminofunktionalisiertes Silikonpolymer (F-1) eingesetzt wurde, dass mindestens eine, bevorzugt mehrere Struktureinheiten der Formel (Si-Amino) besitzt.

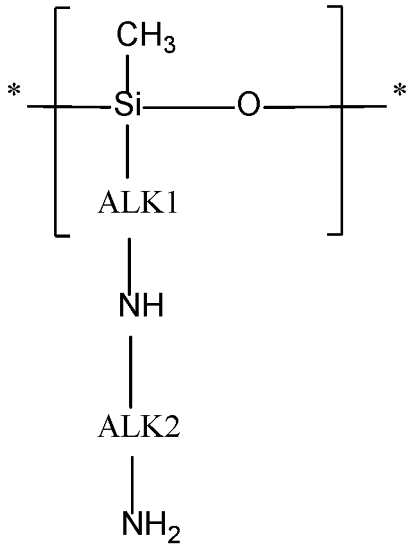

(Si-Amino)

In den Struktureinheiten der Formel (Si-Amino) steht die Kürzel ALK1 und ALK2 unabhängig voneinander für eine lineare oder verzweigte, zweiwertige $C_1$-$C_{20}$-Alkylengruppe.

In einer weiteren ganz besonders bevorzugten Ausführungsform ist ein erfindungsgemäßes Verfahren dadurch gekennzeichnet, dass das Färbemittel (F) mindestens ein aminofunktionalisiertes Silikonpolymer (F-1) enthält, das mindestens eine Struktureinheit der Formel (Si-Amino) umfasst,

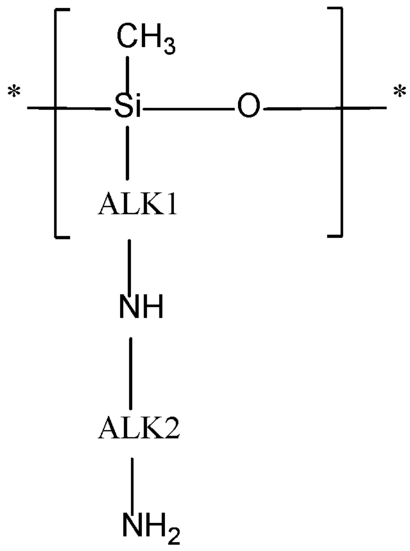

(Si-Amino)

wobei

ALK1 und ALK2 unabhängig voneinander für eine lineare oder verzweigte, zweiwertige $C_1$-$C_{20}$-Alkylengruppe stehen.

Die mit einem Stern (*) gekennzeichneten Position geben hierbei jeweils die Bindung zu weiteren Struktureinheiten des Silikonpolymers an. Beispielsweise kann das dem Stern benachbarte Silicium-Atom an ein weiteres Sauerstoffatom gebunden sein, und das dem Stern benachbarte Sauerstoffatom kann an ein weiteres Siliciumatom oder auch an eine $C_1$-$C_6$-Alkylgruppe gebunden sein.

Eine zweiwertige $C_1$-$C_{20}$-Alkylengruppe kann alternativ auch als eine divalente oder zweibindige $C_1$-$C_{20}$-Alkylengruppe bezeichnet werden, womit gemeint ist, dass jede Gruppierung ALK1 bzw. AK2 zwei Bindungen eingehen kann.

Im Fall von ALK1 erfolgt eine Bindung vom Silicium-Atom zur Gruppierung ALK1, und die zweite Bindung besteht zwischen ALK1 und der sekundären Aminogruppe.

Im Fall von ALK2 erfolgt eine Bindung von der sekundären Aminogruppe zur Gruppierung ALK2, und die zweite Bindung besteht zwischen ALK2 und der primären Aminogruppe.

Beispiele für eine lineare zweiwertige $C_1$-$C_{20}$-Alkylengruppe sind beispielsweise die Methylen-gruppe ($-CH_2-$), die Ethylengruppe ($-CH_2-CH_2-$), die Propylengruppe ($-CH_2-CH_2-CH_2-$) und die Butylengruppe ($-CH_2-CH_2-CH_2-CH_2-$). Die Propylengruppe ($-CH_2-CH_2-CH_2-$) ist besonders bevorzugt. Ab einer Kettenlänge von 3 C-Atomen können zweiwertige Alkylengruppen auch verzweigt sein. Beispiele für verzweigte, zweiwertige $C_3$-$C_{20}$-Alkylengruppen sind ($-CH_2-CH(CH_3)-$) und ($-CH_2-CH(CH_3)-CH_2-$).

In einer weiteren besonders bevorzugten Ausführungsform stellen die Struktureinheiten der Formel (Si-Amino) Wiederholungseinheiten im aminofunktionalisierten Silikonpolymer (F-1) dar, so dass das Silikonpolymer mehrer Struktureinheiten der Formel (Si-Amino) umfasst.

Im Folgenden werden besonders gut geeignete aminofunktionalisierte Silikonpolymere (F-1) mit mindestens einer sekundären Aminogruppe aufgelistet.

Färbungen mit den allerbesten Waschechtheiten konnten erhalten werden, wenn im erfindungsgemäßen Verfahren mindestens ein Färbemittel (F) auf dem keratinischen Material appliziert wurde, das mindestens ein aminofunktionalisiertes Silikonpolymer (F-1) enthält, das Struktureinheiten der Formel (Si-I) und der Formel (Si-II) umfasst

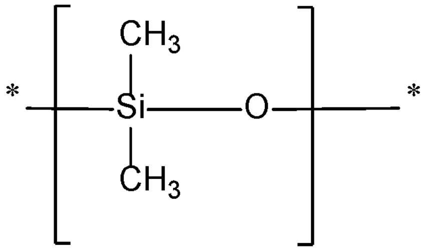

(Si-I)

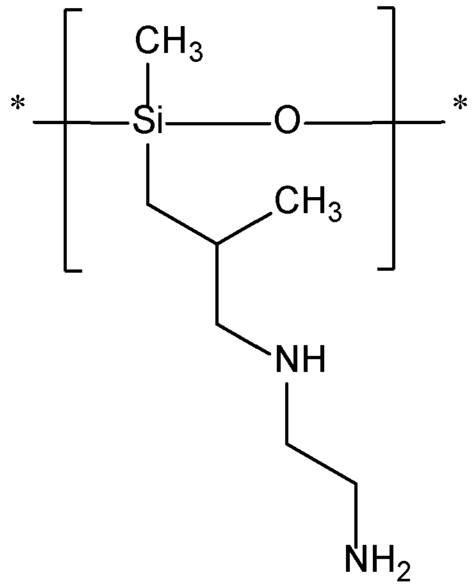

(Si-II).

In einer weiteren explizit ganz besonders bevorzugten Ausführungsform ist ein erfindungsgemäßes Verfahren dadurch gekennzeichnet, dass das Färbemittel (F) mindestens ein aminofunktionalisiertes Silikonpolymer (F-1) enthält, das Struktureinheiten der Formel (Si-I) und der Formel (Si-II) umfasst

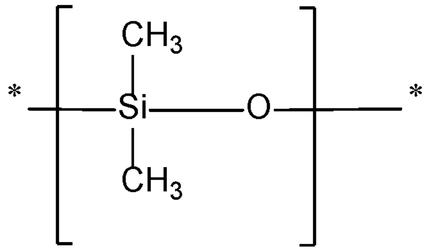

(Si-I)

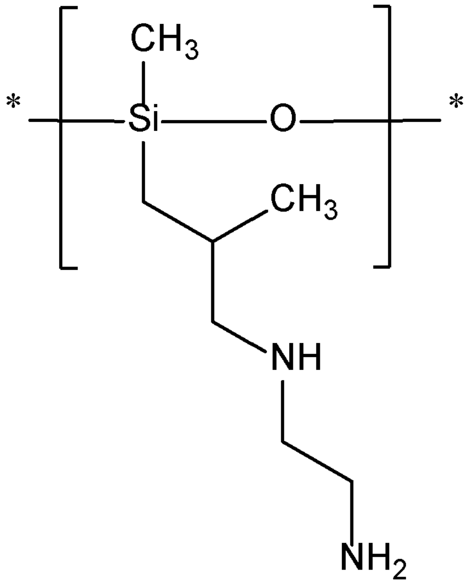

(Si-II).

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt.

Im Rahmen einer weiteren bevorzugten Ausführungsform ist ein erfindungsgemäßes Verfahren gekennzeichnet durch die Anwendung eines Färbemittels (F) auf dem keratinischem Material, wobei das Färbemittel (F) mindestens ein aminofunktionelles Silikonpolymer (F-1) der Formel der Formel (Si-III) enthält,

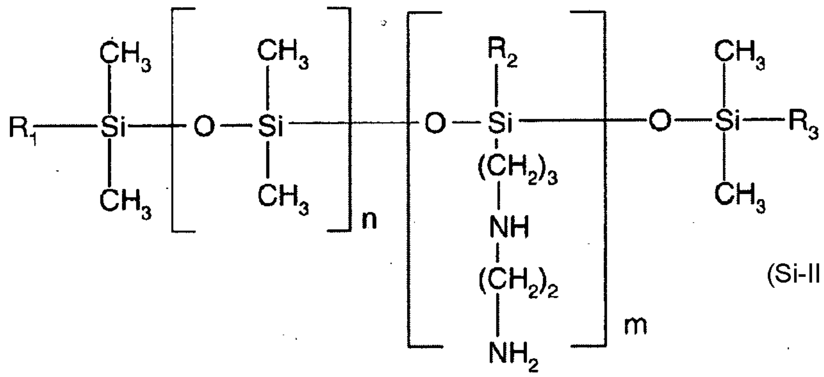

(Si-III)

wobei

- m und n bedeuten Zahlen, die so gewählt sind, daß die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere erfindungsgemäß bevorzugte Verfahren sind gekennzeichnet durch die Anwendung eines Färbemittels (F) auf dem keratinischem Material, wobei das Färbemittel (F) mindestens aminofunktionelles Silikonpolymer (F-1) der Formel der Formel (Si-IV) enthält,

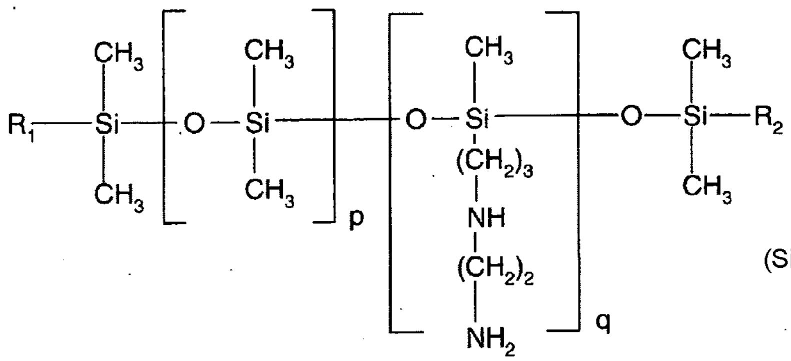

(Si-IV)

in der

- p und q bedeuten Zahlen, die so gewählt sind, daß die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (Si-III) und (Si-IV) unterscheiden sich durch die Gruppierung am Si-Atom, das die stickstoffhaltige Gruppe trägt: In Formel (Si-III) bedeutet R2 eine Hydroxygruppe oder eine C1-4-Alkoxygruppe, während der Rest in Formel (Si-IV) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (Si-III) und (Si-IV) ist nicht zwingend jedes R1-$Si(CH_3)_2$-Gruppe an eine -[O-$Si(CH_3)_{2]}$-Gruppierung gebunden.

Als besonders wirkungsvoll im Hinblick auf die gewünschten Effekte haben sich auch erfindungsgemäßen Verfahren erwiesen, in welchen ein Färbemittel (F) auf den Keratinfasern appliziert wird, welches mindestens ein aminofunktionelles Silikonpolymer (F-1) der Formel der Formel (Si-V) enthält

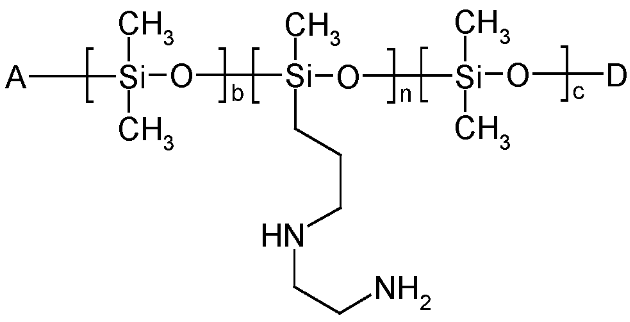

(Si-V),

in der

| | |
|---|---|
| A | für eine Gruppe -OH, -O-$Si(CH_3)_3$,-O-$Si(CH_3)_2OH$ ,-O-$Si(CH_3)_2OCH_3$ steht, |
| D | für eine Gruppe -H, -$Si(CH_3)_3$,-$Si(CH_3)_2OH$, -$Si(CH_3)_2OCH_3$ steht, |
| b, n und c | für ganze Zahlen zwischen 0 und 1000 stehen, mit den Maßgaben |
| | -n > 0 und b + c > 0 |
| | -mindestens eine der Bedingungen A = -OH bzw. D = -H ist erfüllt. |

In der vorstehend genannten Formel (Si-V) sind die einzelnen Siloxaneinheiten mit den Indices b, c und n statistisch verteilt, d.h. es muß sich nicht zwingend um Blockcopolymere handeln.

Das Färbemittel (F) kann weiterhin auch ein oder mehrere verschiedene aminofunktionalisierte Silikonpolymere enthalten, die durch die Formel (Si-VI)

$$M(R_aQ_bSiO_{(4-a-b)/2)x}(RcSiO_{(4-c)/2)y}M \qquad \text{(Si-VI)}$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, $-CH_2CH(CH_3)CH_2-$, Phenylen, Naphthylen, $-CH_2CH_2SCH_2CH_2-$, $-CH_2CH_2OCH_2-$, $-OCH_2CH_2-$, $-OCH_2\ CH_2CH_2-$, $-CH_2CH(CH_3)C(O)OCH_2-$, $-(CH_2)_3\ CC(O)OCH_2CH_2-$, $-C_6H\ _4C_6H_4-$, $-C_6H\ _4CH_2C_6H_4-$; und $-(CH_2)_3C(O)SCH_2CH_2-$ ein.

Z ist ein organischer, aminofunktioneller Rest, enthaltend mindestens eine funktionelle Aminogruppe. Eine mögliche Formel für Z ist $NH(CH_2)_zNH_2$, worin z 1 oder mehr ist. Eine andere mögliche Formel für Z ist $-NH(CH_2)_z(CH_2)_{zz}-NH$, worin sowohl z als auch zz unabhängig 1 oder mehr sind, wobei diese Struktur Diamino-Ringstrukturen umfaßt, wie Piperazinyl. Z ist am bevorzugtesten ein $-NHCH_2CH_2NH_2$-Rest. Eine andere mögliche Formel für Z ist $-N(CH_2)_z(CH_2)_{zz}NX_2$ oder $-NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel $-CH_2CH_2CH_2NHCH_2CH_2NH\ _2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_bSiO_{(4-a-b)/2}$-Einheiten zu den $R_cSiO_{(4c)/2}$-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer besonders bevorzugten Ausführungsform ist ein erfindungsgemäßes Verfahren gekennzeichnet durch die Anwendung eines Färbemittels (F) auf dem keratinischem Material, wobei das Färbemittel (F) ein aminofunktionelles Silikonpolymer der Formel (Si-VII)

$$R'_aG3-_a-Si(OSiG_{2)n}-(OSiG_bR'_{2-b)m}-O-SiG_{3-a}-R'_a \qquad \text{(Si-VII)},$$

enthält, worin bedeutet:

- G ist-H, eine Phenylgruppe, -OH, $-O-CH_3$, $-CH_3$, $-O-CH_2CH_3$, $-CH_2CH_3$, $-O-CH_2CH_2CH_3$,$-CH_2CH_2CH_3$, $-O-CH(CH_3)_2$, $-CH(CH_3)_2$, $-O-CH_2CH_2CH_2CH_3$, $-\ CH_2CH_2CH_2CH_3$, $-O-CH_2CH(CH_3)_2$, $-CH_2CH(CH_3)_2$, $-O-CH(CH_3)CH_2CH_3$, $-\ CH(CH_3)CH_2CH_3$, $-O-C(CH_3)_3$, $-C(CH_3)_3$;
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
    - $-Q-N(R'')-CH_2-CH_2-N(R'')_2$
    - $-Q-N(R'')_2$
    - $-Q-N^+(R'')_3A^-$
    - $-Q-N^+H(R'')_2A^-$
    - $-Q-N^+H_2(R'')A^-$
    - $-Q-N(R'')-CH_2-CH_2-N^+R''H_2A^-$,

wobei jedes Q für eine chemische Bindung, $-CH_2-$, $-CH_2-CH_2-$, $-CH_2CH_2CH_2-$, $-C(CH_3)_2-$ , $-CH_2CH_2CH_2CH_2-$, $-CH_2C(CH_3)_2-$, $-CH(CH_3)CH_2CH_2-$ steht,
R" für gleiche oder verschiedene Reste aus der Gruppe -H, -Phenyl, -Benzyl, $-CH_2-CH(CH_3)Ph$, der $C_{1-20}$-Alkylreste, vorzugsweise $-CH_3$, $-CH_2CH_3$, $-CH_2CH_2CH_3$, $-CH(CH_3)_2$, $-CH_2CH_2CH_2H_3$, $-CH_2CH(CH_3)_2$, $-CH(CH_3)CH_2CH_3$, $-C(CH_3)_3$, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein erfindungsgemäßes Verfahren gekennzeichnet durch die Anwendung eines Färbemittels (F) auf dem keratinischem Material, wobei das Färbemittel (F) mindestens ein aminofunktionelles Silikonpolymer (F-1) der Formel (Si-VIIa) enthält,

$$(CH_3)_3Si-[O-Si(CH_3)_2]_n[OSi(CH_3)]_m-OSi(CH_3)_3 \qquad \text{(Si-VIIa)},$$

$$|$$

$$CH_2CH(CH_3)CH_2NH(CH_2)_2NH_2$$

worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein erfindungsgemäßes Verfahren gekennzeichnet durch die Anwendung eines Färbemittels (F) auf dem keratinischem Material, wobei das Färbemittel (F) mindestens ein aminofunktionelles Silikonpolymer der Formel (Si-VIIb) enthält

$$R-[Si(CH_3)_2-O]_{n1}[Si(R)-O]_m-[Si(CH_3)_2]_{n2}-R \qquad \text{(Si-VIIb)},$$

$$|$$

$$(CH_2)_3NH(CH_2)_2NH_2$$

enthalten, worin R für -OH, -O-CHs oder eine $-CH_3$-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese aminofunktionalisierten Siliconpolymere werden nach der INCI-Deklaration als Amodimethicone bezeichnet.

Unabhängig davon, welche aminofunktionellen Silicone eingesetzt werden, sind erfindungsgemäße Färbemittel (F) bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Weiterhin sind auch Färbemittel (F) zum Einsatz im erfindungsgemäßen Verfahren geeignet, welche ein spezielles 4-Morpholinomethyl-substituiertes Silikonpolymer (F-1) enthielten. Dieses aminofunktionalisierte Silikonpolymer umfasst Struktureinheiten der Formeln (SI-VIII) und der Formel (Si-IX)

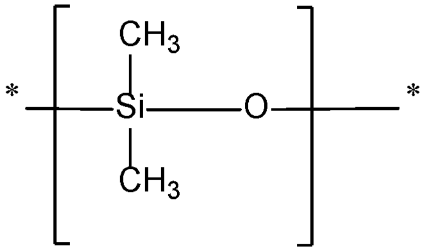

(Si-VIII)

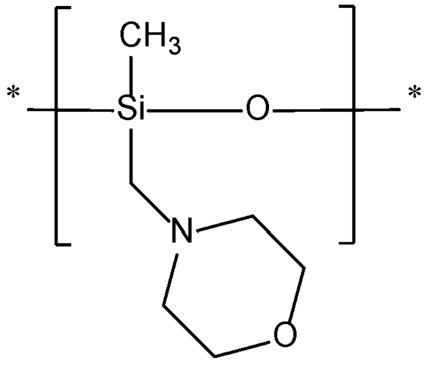

(Si-IX).

Entsprechende 4-Morpholinomethyl-substituiertes Silikonpolymere werden im folgenden beschrieben.

Ein entsprechendes aminofunktionaliserte Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches Struktureinheiten der Formeln (Si-VIII), (Si-IX) und (Si-X) aufweist

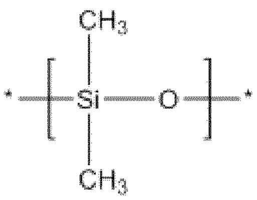
(Si-VIII),
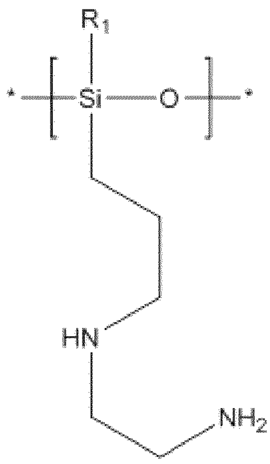
(Si-X)
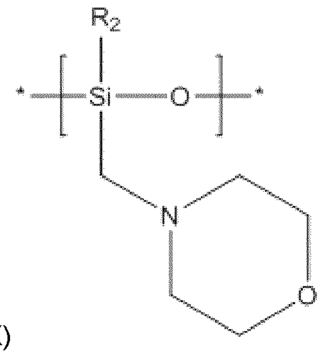
(Si-IX)

in denen

R1 für $-CH_3$, $-OH$, $-OCH_3$, $-O-CH_2CH_3$, $-O-CH_2CH_2CH_3$, oder $-O-CH(CH_3)_2$ steht;
R2 für $-CH_3$, $-OH$, oder $-OCH_3$ steht.

Besonders bevorzugte erfindungsgemäße Färbemittel (F) enthalten mindestens ein 4-morpholinomethyl-substituierten Silikons der Formel (Si-XI)

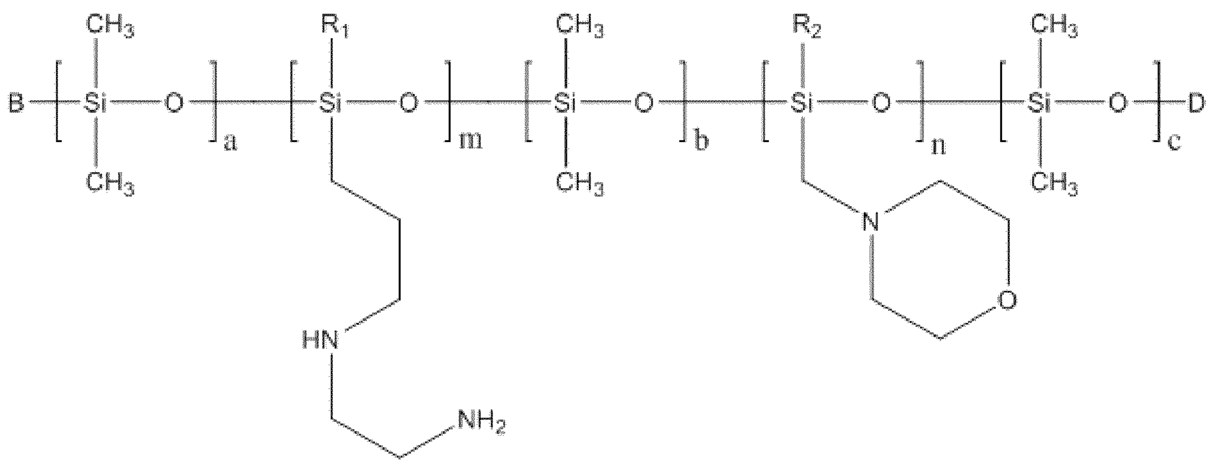

(Si-XI)

in der

R1 für $-CH_3$, $-OH$, $-OCH_3$, $-O-CH_2CH_3$, $-O-CH_2CH_2CH_3$, oder $-O-CH(CH_3)_2$ steht;
R2 für $-CH_3$, $-OH$, oder $-OCH_3$ steht.
B für eine Gruppe $-OH$, $-O-Si(CH_3)_3$,$-O-Si(CH_3)_2OH$ ,$-O-Si(CH_3)_2OCH_3$ steht,
D für eine Gruppe $-H$, $-Si(CH_3)_3$,$-Si(CH_3)_2OH$, $-Si(CH_3)_2OCH_3$ steht,

a, b und c unabhängig voneinander für ganze Zahlen zwischen 0 und 1000 stehen, mit der Maßgabe a + b + c > 0

m und n unabhängig voneinander für ganze, Zahlen zwischen 1 und 1000 stehen
mit den Maßgabe, daß

- mindestens eine der Bedingungen B = -OH bzw. D = -H erfüllt ist,
- die Einheiten a, b, c, m und n statistisch oder blockweise im Molekül verteilt vorliegen.

Strukturformel (Si-XI) soll verdeutlichen, daß die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = $-Si(CH_3)_3$), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

B = $-O-Si(CH_3)_2OH$ und D = $-Si(CH_3)_3$
B = $-O-Si(CH_3)_2OH$ und D = $-Si(CH_3)_2OH$
B = $-O-Si(CH_3)_2OH$ und D = $-Si(CH_3)_2OCH_3$
B = $-O-Si(CH_3)_3$ und D = $-Si(CH_3)_2OH$
B = $-O-Si(CH_3)_2OCH_3$ und D = $-Si(CH_3)_2OH$

bedeutet. Diese Silikone führen zu exorbitanten Verbesserungen der Haareigenschaften der mit den erfindungsgemäßen Mitteln behandelten Haare, und zu einem gravierend verbesserten Schutz bei oxidativer Behandlung.

Es hat sich als besonders vorteilhaft herausgestellt, wenn das erfindungsgemäße Färbemittel das oder die aminofunktionalisierten Silikonpolymere (F-1) in bestimmten Mengenbereichen enhält. Besonders gute Ergebnisse konnten erhalten werden, wenn das Färbemittel - bezogen auf das Gesamtgewicht des Färbemittels - ein oder mehrere aminofunktionalisierte Silikonpolymere (F-1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Im Rahmen einer weiteren besonders bevorzugten Ausführungsform ist ein erfindungsgemäßes Verfahren dadurch gekennzeichnet, dass das Färbemittel (F) - bezogen auf das Gesamtgewicht des Färbemittels - ein oder mehrere aminofunktionalisierte Silikonpolymere (F-1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Pigmente (F-2) im Färbemittel (F)

Als zweiten erfindungswesentlichen Bestandteil enthält das im erfindungsgemäßen Verfahren eingesetzte Färbemittel (F) mindestens ein Pigment.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Färbemittel (F) dadurch gekennzeichnet, dass es mindestens eine farbgebende Verbindung (F-2) aus der Gruppe der anorganischen und/oder organischen Pigmente enthält.

In einer bevorzugten Ausführungsform ist ein erfindungsgemäßes Färbemittel (F) dadurch gekennzeichnet, dass es mindestens ein anorganisches und/oder organisches Pigment (F-2) enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Verfahren dadurch gekennzeichnet, dass das Färbemittel (F) mindestens eine farbgebende Verbindung (F-2) aus der Gruppe der anorganischen Pigmente enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (a) mindestens eine farbgebende Verbindung (F-2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)

Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491 (Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491 (Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch (a) ein oder mehrere farbgebende Verbindungen (F-2) aus der Gruppe der organischen Pigmente enthalten
Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Verfahren dadurch gekennzeichnet, dass das Färbemittel (F) mindestens eine farbgebende Verbindungen (F-2) aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Färbemittel (F) des erfindungsgemäßen Verfahrens ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße $D_{50}$ von 1,0 bis 50 $\mu$m, vorzugsweise von 5,0 bis 45 $\mu$m, bevorzugt von 10 bis 40 $\mu$m, insbesondere von 14 bis 30 $\mu$m, aufweist. Die mittlere Teilchengröße $D_{50}$ kann beispielsweise unter Verwendung von dynamischer Lichtstreuung (DLS) bestimmt werden.

Das bzw. die Pigmente (F-2) stellen den zweiten wesentlichen Bestandteil des erfindungsgemäßen Färbemittels dar und werden bevorzugt in bestimmten Mengenbereichen im Mittel eingesetzt. Besonders gute Ergebnisse wurden erhalten, wenn das Färbemittel - bezogen auf das Gesamtgewicht des Färbemittels - ein oder mehrere Pigmente (F-2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthielt.

In einer weiteren ganz besonders bevorzugten Ausführungsform ist ein erfindungsgemäßes Verfahren dadurch gekennzeichnet, dass das Färbemittel (F) - bezogen auf das Gesamtgewicht des Färbemittels - ein oder mehrere Pigmente (F-2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

Als optionale Bestandteile können die im erfindungsgemäßen Verfahren angewendeten Färbemittel (F) auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L.

Direktziehende Farbstoffe können in anionische, kationische und nichtionische direktziehende Farbstoffe unterteilt werden.

In einer weiteren Ausführungsform ist ein erfindungsgemäßes Verfahren dadurch gekennzeichnet, dass das Färbemittel (F) zusätzlich mindestens einen anionischen, nichtionischen und kationischen direktziehenden Farbstoffe enthält.

Geeignete kationische direktziehende Farbstoffe sind beispielsweise Basic Blue 7, Basic Blue 26, HC Blue 16, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76.

Als nichtionische direktziehende Farbstoffe können beipsielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol,1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Im Zuge der zu dieser Erfindung führenden Arbeiten hat sich herausgestellt, dass auch mit Mitteln (F) die mindestens einen anionischen direktziehenden Farbstoff (F-2) enthalten, Färbungen mit guten Farbintensitäten und Echtheiten erzeugt werden können.

In einer weiteren Ausführungsform ist ein erfindungsgemäßes Verfahren daher dadurch gekennzeichnet, dass das Färbemittel (F) mindestens einen anionischen direktziehenden Farbstoff enthält.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung ($-SO_3H$) besitzen. In Abhängigkeit vom pH-Wert liegen die protonierten Formen (-COOH, $-SO_3H$) der Carbonsäure- bzw. Sulfonsäuregruppierungen mit ihren deprotonierten Formen ($-COO^-$, $-SO_3^-$ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden

Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer weiteren Ausführungsform ist ein Verfahren zum Färben von keratinischem Material dadurch gekennzeichnet ist, dass das Färbemittel (F) mindestens einen anionischen direktziehenden Farbstoff enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe ($-SO_3H$) eine Natriumsulfonatgruppe (-SOsNa) und/oder eine Kaliumsulfonatgruppe ($-SO_3K$) besitzen.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n°: C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intenstität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1 ,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

In einer weiteren Ausführungsform ist ein erfindungsgemäßes Verfahren ist daher dadurch gekennzeichnet, dass das Färbemittel (F) mindestens einen direktziehenden Farbstoff (F-2) enthält, der ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe können je nach erwünschter Farbintensität in verschiedenen Mengen im Färbemittel (F) eingesetzt werden. Besonders gute Ergebnisse konnten erhalten werden, wenn das Färbemittel (F) - bezogen auf das Gesamtgewicht des Färbemittels (F) - ein oder mehrere direktziehende Farbstoffe (b) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

Weiterhin kann das Färbemittel (F) als farbgebende Verbindung (F-2) auch mindestens einen photochromen oder thermochromen Farbstoff enthalten.

Photochrome Farbstoffe sind Farbstoffe, die auf Bestrahlung mit UV-Licht (Sonnenlicht oder Schwarzlicht) mit einer reversiblen Farbtonänderung reagieren. Dabei verändert das UV-Licht die chemische Struktur der Farbstoffe und damit ihr Absorptionsverhalten (Photochromie).

Thermochrome Farbstoffe sind Farbstoffe, die auf Temperaturänderungen mit einer reversiblen Farbtonänderung reagieren. Dabei verändert die Temperaturänderung die chemische Struktur der Farbstoffe und damit ihr Absorptionsverhalten (Thermochromie).

Das Färbemittel (F) kann - bezogen auf das Gesamtgewicht des Färbemittels (F) - ein oder mehrere photochrome Farbstoffe (b) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthalten

Das Färbemittel (F) kann - bezogen auf das Gesamtgewicht des Färbemittels (F) - ein oder mehrere thermochrome Farbstoffe (b) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthalten

<u>weitere optionale Inhalttstoffe in den Mitteln (V) und/oder (F)</u>

Zusätzlich zu den bereits beschriebenen erfindungswesentlichen Bestandteilen können das Vorbehandlungsmittel (V) und/oder das Färbemittel (F) auch noch weitere optionale Inhaltsstoffe enthalten.

So können die Mittel ferner auch noch weitere Wirk-, Hilfs- und Zusatzstoffe enthalten, wie beispielsweise Lösungsmittel, Fettbestandteile wie beispielsweise der $C_8$-$C_{30}$-Fettalkohole, der $C_8$-$C_{30}$-Fettsäuretriglyceride, der $C_8$-$C_{30}$-Fettsäuremonoglyceride, der $C_8$-$C_{30}$-Fettsäurediglyceride und/oder der Kohlenwasserstoffe; Polymere; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, $N_2O$, Dimethylether,

$CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

Der Sinn des Vorbehandlungsmittels (V) besteht jedoch in der Entfernung aller Substanzen, die sich durch eine zuvor erfolgte Anwendung eines Conditioners oder Stylingmittels auf der Oberfläche des Keratinmaterial abgelagert haben. Hierbei wird vermutet, dass insbesondere Polymere und/oder kationische Tenside aufgrund ihrer hohen Affinität zum Keratinmaterial eine negative Einfluss auf ein später angewendetes Pigment-Färbemittel ausüben können.

Aus diesem Grund ist es ganz besonders bevorzugt, im erfindungsgemäßen Vorbehandlungsmittel (V) keine Polymere und keine kationischen Tenside einzusetzen oder aber deren Einsatzmengen stark zu minimieren.

Im Rahmen einer weiteren bevorzugtenAusführungsform ist ein erfindungsgemäßes Verfahren dadurch gekennzeichnet, dass der Gesamtgehalt aller im Vorbehandlungsmittel (V) enthaltenen Polymere - bezogen auf das Gesamtgewicht des Vorbehandlungsmittels - unterhalb von 0,5 Gew.-%, bevorzugt unterhalb on 0,1 Gew.-% und ganz besonders bevorzugt unterhalb von 0,01 Gew.-% liegt.

Im Rahmen einer weiteren bevorzugten Ausführungsform ist ein erfindungsgemäßes Verfahren dadurch gekennzeichnet, dass der Gesamtgehalt aller im Vorbehandlungsmittel (V) enthaltenen kationischen Tenside - bezogen auf das Gesamtgewicht des Vorbehandlungsmittels - unterhalb von 0,5 Gew.-%, bevorzugt unterhalb on 0,1 Gew.-% und ganz besonders bevorzugt unterhalb von 0,01 Gew.-% liegt.

pH-Wert von Vorbehandlungsmittel (V) und/oder Färbemittel (F)

Die pH-Werte der erfindungsgemäßen Mittel (V) und (F) können auf einen leicht sauren bis alkalischen pH-Wert eingestellt werden.

Im Rahmen einer Ausführungsform besitzt das Vorbehandlungsmittel (V) einen pH-Wert von 2,0 bis 6,5, bevorzugt von 2,5 bis 6,0, weiter bevorzugt von 3,0 bis 5,5 und ganz besonders bevorzugt von 3,5 bis 5,0. Wurden Vorbehandlungsmittel (V) mit diesen pH-Werten im erfindungsgemäßen Verfahren eingesetzt, so ließen sich Färbungen mit besonders hoher Farbintensität erzielen.

Im Rahmen einer weiteren Ausführungsform besitzt das Vorbehandlungsmittel (V) einen pH-Wert von 7,0 bis 11,5, bevorzugt von 7,5 bis 11,0 und ganz besonders bevorzugt von 8,0 bis 10,5. Wurden Vorbehandlungsmittel (V) mit diesen pH-Werten im erfindungsgemäßen Verfahren eingesetzt, so ließen sich Färbungen mit besonders guter Gleichmäßigkeit erzielen.

Die pH-Werte des erfindungsgemäßen Färbemittels (F) können auf einen leicht sauren bis alkalischen pH-Wert eingestellt werden. Ganz besonders bevorzugt besitzt das Färbemittel (F) einen pH-Wert im Bereich von 5,0 bis 10,0, bevorzugt von 6,0 bis 9,5, weiter bevorzugt von 6,0 bis 8,7 und ganz besonders bevorzugt von 6,0 bis 7,5.

Zur Einstellung der jeweiligen gewünschten pH-Werte können die dem Fachmann bekannten Alkalisierungsmittel und Acidifizierungsmittel verwendet werden. Bei den pH-Werten im Sinne der vorliegenden Erfindung handelt es sich um pH-Werte, die bei einer Temperatur von 22°C gemessen wurden.

Als Alkalisierungsmittel können die Mittel beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -SOsH-Gruppe enthält. Bevorzugte Aminosäuren sind Aminocarbonsäuren, insbesondere α,-(alpha)-Aminocarbonsäuren und ω-Aminocarbonsäuren, wobei α,-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α,-Aminocarbonsäuren enthalten mindestens ein asymmetrisches Kohlenstoffatom. Im Rahmen der

vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ebenfalls erfindungsgemäß ist die Einstellung des gewünschten pH-Werte über ein Puffersystem. Unter einem Puffer oder einem Puffersystem wird üblicherweise ein Gemisch aus einer schwachen oder mittelstarken Säure (z. B. Essigsäure) mit einem praktisch völlig dissoziierten Neutralsalz derselben Säure (z. B. Natriumacetat) verstanden. Wird etwas Base oder Säure zugegeben, so ändert sich der pH-Wert kaum (Pufferung). Die Wirkung der in einer Pufferlösung enthaltenen Puffersubstanzen beruht auf der Abfangreaktion von Wasserstoff- bzw. Hydroxid-Ionen unter Bildung schwacher Säuren bzw. Basen aufgrund ihres Dissoziationsgleichgewichts.

Ein Puffersystem kann aus einem Gemisch einer anorganischen oder organischen Säure und einem korrespondierenden Salz dieser Säure ausgebildet werden.

Säuren können gepuffert werden durch alle Salze aus schwachen Säuren und starken Basen, Basen durch Salze aus starken Säuren und schwachen Basen. Die starke (vollständig in Ionen dissoziierte) Salzsäure kann z. B. durch Zusatz von Natriumacetat abgepuffert werden. Entsprechend dem Gleichgewicht

$$H_3C{-}COONa + HCl \rightleftharpoons NaCl + H_3C{-}COOH$$

wird Salzsäure durch Natriumacetat unter Bildung von Kochsalz in die schwache Essigsäure übergeführt, die in Gegenwart eines Natriumacetat-Überschusses nur zu einem sehr geringen Anteil dissoziiert. Puffer, die sowohl gegenüber Säuren als auch Basen wirken, sind Gemische aus schwachen Säuren und ihren Salzen.

Literaturbekannte Beispiele für Puffersysteme sind Essigsäure/Natriumacetat, Borsäure/Natriumborat, Phosphorsäure/Natriumphosphat und Hydrogencarbonat/Soda.

Der pH-Wert des erfindungsgemäßen Mittels kann zum Beispiel durch Zusatz eines anorganischen oder organischen Puffersystems eingestellt werden. Unter einem anorganischen Puffersystem wird im Sinne der vorliegenden Erfindung eine Mischung aus einer anorganischen Säure und ihrer konjugierten korrespondieren anorganischen Base verstanden.

Unter einem organischen Puffersystem wird im Sinne der vorliegenden Erfindung eine Mischung aus einer organischen Säure und ihrer konjugierten korrespondieren Base verstanden. Bedingt durch den organischen Säure-Rest ist auch die konjugierte korrespondierende Base der organischen Säure ebenfalls organisch. Hierbei kann das zur Neutralisation der Ladung des Säure-Anions vorhandene Kation anorganisch oder organisch sein.

Beispiele für anorganische Säuren sind Schwefelsäure, Salzsäure und Phosphorsäure ($H_3PO_4$). Bei Phosphorsäure handelt es sich um eine mittelstarke Säure, die ganz besonders bevorzugt ist.

Eine ganz besonders gut geeignete anorganische Säure ist Kaliumdihydrogenphosphat Kaliumdihydrogenphosphat besitzt die Summenformel $KH_2PO_4$ und trägt die CAS-Nummer 7778-77-0. Kaliumdihydrogenphosphat besitzt eine molare Masse von 136,09 g/mol. Es ist gut löslich in Wasser (222 g/l bei 20 °C) und reagiert in Wasser sauer. Eine 5 %ige Lösung von Kaliumdihydrogenphosphat in Wasser besitzt einen pH-Wert von 4,4.

Eine weitere ganz besonders gut geeignete anorganische Säure ist Natriumdihydrogenphosphat. Natriumdihydrogenphosphat besitzt die Summenformel $NaH_2PO_4$ und trägt die CAS-Nummern 7558-80-7 (Anhydrat), 10049-21-5 (Monohdrat) und 13472-35-0 (Dihydrat). Das wasserfreie Natriumdihydrogenphosphat besitzt eine molare Masse von 119,98 g/mol. Natriumdihydrogenphosphat reagiert in wässriger Lösung sauer.

Besonders bevorzugt als korrespondierendes Salz der vorgenannten beiden Säuren ist Dikaliumhydrogenphosphat. Dikaliumhydrogenphosphat besitzt die Summenformel $K_2HPO_4$ und trägt die CAS-Nummern 7758-11-4 (wasserfrei) und 16788-57-1 (Trihydrat). Das wasserfreie Dikaliumhydrogenphosphat besitzt eine molare Masse von 174,18 g/mol. Dikaliumhydrogenphosphat reagiert in wässriger Lösung alkalisch.

Ebenfalls besonders bevorzugt als korresprondierdens Salz der vorgenannten beiden Säuren ist Dinatriumhydrogenphosphat. Dinatriumhydrogenphosphat besitzt die Summenformel $Na_2HPO_4$ und trägt die CAS-Nummern 7558-79-4 (wasserfrei), 10028-24-7 (Dihydrat), 7782-85-6 (Heptahydrat) und 10039-32-4 (Dodecahydrat). Das wasserfreie Dinatriumhydrogenphosphat besitzt eine molare Masse von 141,96 g/mol. Dinatriumhydrogenphosphat reagiert in wässriger Lösung alkalisch.

Beispiele für organische Säuren sind Zitronensäure, Bernsteinsäure, Weinsäure, Milchsäure, Essigsäure, Äpfelsäure, Malonsäure und Maleinsäure.

Beispiele für die korrespondierenden Salze dieser organischen Säuren sind die Natrium- und Kaliumsalze der Zitronensäure, die Natrium- und Kaliumsalze der Bernsteinsäure, die Natrium- und Kaliumsalze der Weinsäure, die Natrium- und Kaliumsalze der Milchsäure, die Natrium- und Kaliumsalze der Essigsäure, die Natrium- und Kaliumsalze der Äpfelsäure, die Natrium- und Kaliumsalze der Malonsäure und die die Natrium- und Kaliumsalze der Maleinsäure.

Abfolge der Verfahrensschritte

Wie bereits zuvor beschrieben, wird das Vorbehandlungsmittel (V) vor der Anwendung des Färbemittels (F) appliziert. In diesem Zusammenhang hat es sich als ganz besonders bevorzugt herausgestellt, dass Vorbehandlungsmittel (V) auf dem Keratinmaterial anzuwenden, für einen bestimmten Zeitraum einwirken zu lassen und danach wieder mit Wasser auszuspülen.

Ein weiterer Gegenstand ist daher ein Verfahren zur Färbung von keratinischen Fasern, insbesondere menschlichen Haaren, umfassend die folgenden Schritte:

(1) Applizieren eine Vorbehandlungsmittels (V) auf die keratinischen Fasern, wobei das Vorbehandlungsmittel (V) bei der Beschreibung des ersten Erfindungsgegenstands im Detail offenbart wurde,
(2) Einwirken des in Schritt (1) applizierten Vorbehandlungsmittels auf die keratinischen Fasern,
(3) Ausspülen des Vorbehandlungsmittels mit Wasser,
(4) Applizieren eines Färbemittels (F) auf die keratinischen Fasern, wobei das Färbemittel bei der Beschreibung des ersten Erfindungsgegenstands im Detail offenbart wurde,
(5) Einwirken des in Schritt (4) applizierten Färbemittels auf die keratinischen Fasern, und
(6) Ausspülen des Färbemittels mit Wasser, dadurch gekennzeichnet, dass zwischen den Schritten (3) und (4) ein Zeitraum von maximal 3 Stunden liegt

In Schritt (1) des erfindungsgemäßen Verfahrens wird ein Vorbehandlungsmittel (V), das in einem wasserhaltigen Träger mindestens ein anionisches Tensid enthält, auf die Haare appliziert.

Im darauffolgenden Schritt wird das zuvor applizierte Vorbehandlungsmittel (V) auf die Keratinasern einwirken gelassen. In diesem Zusammenhang sind verschiedene Einwirkzeiten von beispielsweise 30 Sekunden bis 60 Minuten denkbar. Bevorzugt sind Einwirkzeiten von 30 Sekunden bis 5 Minuten. Im Anschluss an das Einwirken des Vorbehandlungsmittels (V) auf die Keratinfasern wird dieses schließlich in Schritt (3) mit Wasser ausgespült. Hierbei wird das Vorbehandlungsmittels (V) in einer bevorzugten Ausführungsform nur mit Wasser, d.h. ohne Zuhilfenahme eines nicht erfindungsgemäßen Nachbehandlungsmittels oder eines Shampoos, ausgewaschen.

Im Prinzip kann der Anwender nun den Zeitraum, der zwischen der Anwendung der beiden Mittel (V) und (F) liegt, frei wählen. wobei zwischen den Schritten (3) und (4) ein Zeitraum von maximal 3 Stunden liegt.

Das Färbemittel (F) muss jedoch auf Keratinmaterial bzw. Haaren angewendet werden, die sich in einem Zustand befinden, wie er nach dem Abspülen des Vorbehandlungsmittels (V) vorliegt. Aus diesem Grund ist es erfindungswesentlich, dass zwischen der Anwendung der beiden Mittel (V) und (F) keine weiteren Mittel, wie beispielsweise andere Conditioner, Stylingmittel appliziert werden. Auf diese Weise wird der maximale Zeitraum, der zwischen der Anwendung der beiden Mittel (V) und (F) liegt, auf ein zeitliches Intervall von maximal 3 Stunden begrenzt.

Es hat sich als bevorzugt herausgestellt, wenn zwischen dem Ausspülen des Vorbehandlungsmittels (V) mit Wasser und dem Applizieren eines Färbemittels (F) auf die keratinischen Fasern ein Zeitraum von maximal 3 Stunden liegt.

Im Rahmen einer weiteren bevorzugten Ausführungsform ist ein erfindungsgemäßes Verfahren dadurch gekennzeichnet, dass Schritt (4) direkt im Anschluss an Schritt (3) erfolgt. Mit Schritt (4) erfolgt die Anwendung des Färbemittels.

Das Einwirken des Färbemittels (F) auf den keratinischen Fasern in Schritt (5) kann beispielsweise für einen Zeitraum von 15 Sekunden bis 30 Minuten, bevorzugt für einen Zeitraum von 30 Sekunden bis 15 Minuten, besonderes bevorzugt für einen Zeitraum von 1 bis 15 Minuten, erfolgen.

Danach wird das Färbemittel (F) schließlich in Schritt (6) mit Wasser ausgespült. Hierbei wird das Färbemittel (F) in einer bevorzugten Ausführungsform nur mit Wasser, d.h. ohne Zuhilfenahme eines nicht erfindungsgemäßen Nachbehandlungsmittels oder eines Shampoos, ausgewaschen werden.

Mehrkomponenten-Verpackungseinheit

Zur Erhöhung des Anwenderkomforts werden dem Anwender bevorzugt alle benötigten Mittel in Form einer Mehrkomponenten-Verpackungseinheit (Kit-of-parts) zur Verfügung gestellt.

Diese Mehrkomponenten-Verpackungseinheit (Kit-of-parts) zum Färben von keratinischem Material, umfassend getrennt voneinander konfektioniert, enthält:

- einen ersten Container mit einem Vorbehandlungsmittel (V), wobei das Vorbehandlungsmittel (V) bei der Beschreibung des ersten Erfindungsgegenstands im Detail offenbart wurde, und
- einen zweiten Container mit einem Färbemittel (F), wobei das Färbemittel (F) bei der Beschreibung des ersten Erfindungsgegenstands im Detail offenbart wurde,

Beispiele

1. Formulierungen

Es wurden die folgenden Formulierungen hergestellt (alle Angaben, sofern nichts anderes angegeben ist, in Gew.-%):

| Conditioner | (C) |
|---|---|
| Isopropylmyristat | 0,8 |
| Cetearylalkohol | 3,4 |
| [3-(Behenoyloxy)-2-hydroxypropyl]trimethylammonium chloride (INCI: BEHENOYL PG-TRIMONIUM CHLORIDE) | 1,0 |
| Behentrimoniumchlorid | 0,5 |
| Zitronensäure | 0,5 |
| Methylpararben, Na-Salz | 0,3 |
| Glycerin | 0,2 |
| Cosmedia Triple C (BASF, Polyquaternium-37, Dicaprylyl Carbonate, Lauryl Glucoside) | 0,2 |
| Polyvinylpyrrolidon/Vinylacetat Copolymer (60/40) | 1,0 |
| Phenxoyethanol | 0,4 |
| Parfum | 0,4 |
| Wasser | ad 100 |

| Vorbehandlungsmittel (V) | (V1) | (V2) |
|---|---|---|
| Texapon K 12 G Ecocert Natriumlaurylsulfat (BASF, CAS-Nr. 151-21-3) | --- | 10,0 |
| Natrium laureth sulfat (Natriumlaurylethersulfat, ethoxyliert mit 2 EO) CAS-Nr. 68891-38-3 | 8,5 | --- |
| Cocoamidopropylbetain | 1,5 | --- |
| Ammoniak/Zitronensäure | ad pH 4,6 | ad pH 10,0 |
| Wasser | ad 100 | ad 100 |

| Färbemittel (F) | F |
|---|---|
| Cetylalkohol | 3,0 g |
| Stearylalkohol | 3,0 g |
| Ceteareth-30, (Cetearylalkohol, ethoxylated 30 EO) | 1,5 g |
| Kaliumdihydrogenphosphat | 0,07 |
| Dinatriumhydrogenphosphat | 0,15 |

(fortgesetzt)

| Färbemittel (F) | F |
|---|---|
| Unipure Red LC3071, organisches Pigment CI 15850 | 1,0 g |
| 1,2-Propandiol | 10,0 g |
| Dow Corning 2-8566 (Siloxanes and Silicones, 3-[(2-Aminoethyl)amino]-2-methylpropyl Me, Di-Me-Siloxane" | 1,0 g |
| Wasser | ad 100 |

2. Anwendung auf Strähnen

Zunächst wurden Haarsträhnen (Firma Kerling) mit dem Condioner behandelt. Hierzu wurde der Conditioner (jeweils 0,2 g pro g Haar) auf das angefeuchtete Haar appliziert, für einen Zeitraum von 5 Minuten einwirken gelassen und anschließend mit Wasser ausgespült.

Auf die noch feuchten Strähnen wurde das jeweilige Vorbehandlungsmittel (V) appliziert. Hierzu wurden jeweils 0,2 g Vorbehandlungsmittel (V) pro g Haar auf die Strähnen gegeben, für 30 Sekunden einmassiert und anschließend mit Wasser ausgespült.

Eine Referenzsträhne wurde ohne Anwendung des Vorbehandlungsmittels (V) direkt mit dem Färbemittel (F) behandelt.

Auf die noch feuchten Haare wurde direkt im Anschluss daran das Färbemittel (F) appliziert. Hierzu wurden jeweils 0,2 g Färbemittel (F) pro g Haarsträhne einmassiert, für 5 Minuten einwirken gelassen und anschließend wieder mit Wasser ausgespült.

Nach Abwschluss der Behandlung wurden die Haarsträhnen farbmetrisch mit einem Farbmessgerät der Firma Datacolor, Typ Spectraflash 450 vermessen.

Der für die Beurteilung der unterschiedlichen Farbintensitäten herangezogene dE-Wert ergibt sich aus den am jeweiligen Strähnenteil gemessenen L*a*b*-Farbmesswerten wie folgt:

$$dE = [ (L_i - L_0)^2 + (a_i - a_0)^2 + (b_i - b_0)]^{1/2}$$

$L_0$, $a_0$ und $b_0$ = Messwerte der Referenzsträhne
$L_i$, $a_i$ und $b_i$ = Messwerte der mit dem erfindungsgemäßen Verfahren erhaltenen Färbung

| Bsp. | Conditioner | Vorbehandlungsmittel | Färbemittel | L | a | b | dE |
|---|---|---|---|---|---|---|---|
| 1 | (C) | --- | (F) | 31,70 | 30,44 | 11,41 | --- |
| 2 | (C) | (V1) | (F) | 21,96 | 31,02 | 11,73 | 9,8 |
| 3 | (C) | (V2) | (F) | 26,24 | 32,30 | 10,06 | 5,9 |

3. Anwendung im Haarstudio

Bei 3 Probanden wurde zunächst der Conditioner appliziert. Hierzu wurde der Conditioner auf das angefeuchtete Haar appliziert, für einen Zeitraum von 5 Minuten einwirken gelassen und anschließend mit Wasser ausgespült.

Dann wurden auf die noch feuchten Haare von zwei Probanden jeweils eines der beiden Vorbehandlungsmittel (V1) und (V2) appliziert, für 30 Sekunden einmassiert und anschließend mit Wasser ausgespült.

Bei einem Probanden wurde kein Vorbehandlungsmittel (V) angewendet.

Danach wurde jeder Proband mit dem Färbemittel (F) behandelt. Hierzu wurde das Färbemittel (F) in die Haare einmassiert für 5 Minuten einwirken gelassen und anschließend wieder mit Wasser ausgespült.

Die Intensität und die Gleichmäßigkeit der Färbung wurden visuell durch geschulte Personen begutachtet.

| Proband | Conditioner | Vorbehandlungsmittel | Färbemittel | Farbintensität | Gleichmäßigkeit |
|---|---|---|---|---|---|
| 1 | (C) | --- | (F) | + | + |

(fortgesetzt)

| Proband | Conditioner | Vorbehandlungsmittel | Färbemittel | Farbintensität | Gleichmäßigkeit |
|---|---|---|---|---|---|
| 2 | (C) | (V1) | (F) | +++ | ++ |
| 3 | (C) | (V2) | (F) | ++ | +++ |

+++ = sehr gut   ++ = mittel   + = schlecht

## Patentansprüche

1. Verfahren zum Färben von keratinischen Fasern, insbesondere menschlichen Haaren, umfassend die folgenden Schritte in der angegebenen Reihenfolge:

   (1) Applizieren eines Vorbehandlungsmittels (V) auf die keratinischen Fasern, wobei das Vorbehandlungsmittel in einem wasserhaltigen Träger
   (V-1) mindestens ein anionisches Tensid enthält,
   (2) Einwirken des in Schritt (1) applizierten Vorbehandlungsmittels auf die keratinischen Fasern,
   (3) Ausspülen des Vorbehandlungsmittels mit Wasser,
   (4) Applizieren eines Färbemittels (F) auf die keratinischen Fasern, wobei das Färbemittel

      (F-1) mindestens ein aminofunktionalisiertes Silikonpolymer und
      (F-2) mindestens ein Pigment enthält,

   (5) Einwirken des in Schritt (4) applizierten Färbemittels auf die keratinischen Fasern, und
   (6) Ausspülen des Färbemittels mit Wasser,

   **dadurch gekennzeichnet, dass** zwischen den Schritten (3) und (4) ein Zeitraum von maximal 3 Stunden liegt.

2. Verfahren nach Anspruch 1, **dadurch gekennzeichnet, dass** das Vorbehandlungsmittel (V) (V-1) mindestens ein anionisches Tensid der Formel (T-1) enthält,

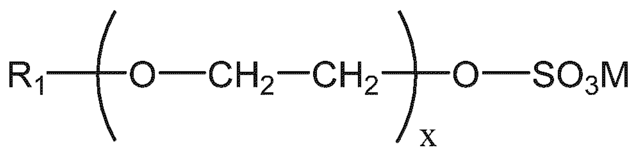

(T-1),

   wobei

   $R^1$ für eine lineare oder verzweigte, gesättigte oder ungesättigte $C_8$-$C_{30}$-Alkylgruppe steht,
   x für eine ganze Zahl von 0 bis 50 steht, und
   M für ein Wasserstoffatom, für Ammonium ($NH_4$), oder für ein Äquivalent eines ein oder mehrwertigen Kations steht.

3. Verfahren nach Anspruch 2, **dadurch gekennzeichnet, dass** das Vorbehandlungsmittel (V) mindestens ein anionisches Tensid (V-1) der Formel (T-1) enthält, wobei

   R für eine lineare, gesättigte oder ungesättigte $C_{12}$-$C_{18}$-Alkylgruppe steht,
   x für eine ganze Zahl von 0 bis 5, bevorzugt für eine ganze Zahl von 1 bis 5, steht,
   M für ein Wasserstoffatom oder für ein Alkalimetallkation, bevorzugt für ein Natriumkation oder für ein Kaliumkation, steht.

4. Verfahren nach einem der Ansprüche 1 bis 3, dadurchgekennzeichnet, dass das Vorbehandlungsmittel (V) - bezogen

auf das Gesamtgewicht des Vorbehandlungsmittels - ein oder mehrere anionische Tenside (V-1) in einer Gesamtmenge von 2,0 bis 18,0 Gew.-%, bevorzugt von 4,0 bis 16,0 Gew.-%, weiter bevorzugt von 6,0 bis 14,0 Gew.-% und ganz besonders bevorzugt von 8,0 bis 12,0 Gew.-% enthält.

**5.** Verfahren nach einem der Ansprüche 1 bis 4, **dadurch gekennzeichnet,dass** das Vorbehandlungsmittel (V) (V-2) mindestens ein zwitterionisches und/oder amphoteres Tensid enthält.

**6.** Verfahren nach Anspruch 5, **dadurch gekennzeichnet,dass** das Vorbehandlungsmittel (V) (V-2) mindestens ein zwitterionisches Tensid enthält, das ausgewählt ist aus den Tensiden der Formel (T-2), (T-3), (T-4) und/oder (T-5),

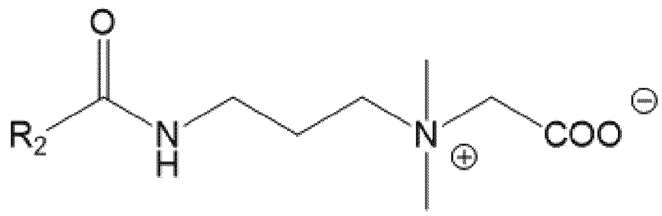

(T-2)

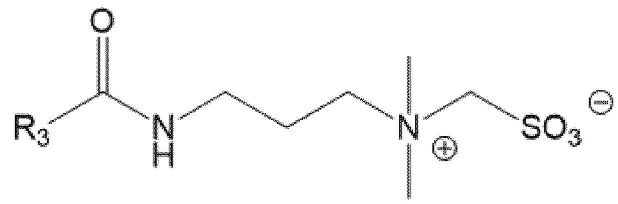

(T-3)

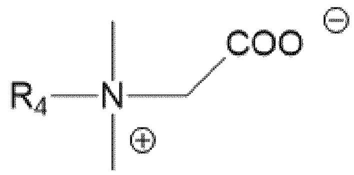

(T-4)

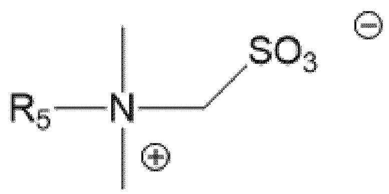

(T-5)

wobei

R2, R3, R4, R5 unabhängig voneinander für eine lineare oder verzweigte, gesättigte oder ungesättigte $C_8$-$C_{30}$-Alkylgruppe, bevorzugt für eine lineare, gesättigte oder ungesättigte $C_{12}$-$C_{18}$-Alkylgruppe, stehen.

**7.** Verfahren nach einem der Ansprüche 1 bis 6, dadurchgekennzeichnet, dass das Vorbehandlungsmittel (V) - bezogen auf das Gesamtgewicht des Vorbehandlungsmittels - ein oder mehrere zwitterionische und/oder amphotere Tenside (V-2) in einer Gesamtmenge von 0,5 bis 8,5 Gew.-%, bevorzugt 1,0 bis 7,5 Gew.-%, weiter bevorzugt 1,5 bis 6,5 Gew.-% und ganz besonders bevorzugt 2,0 bis 4,5 Gew.-% enthält.

8. Verfahren nach einem der Ansprüche 1 bis 7, **dadurch gekennzeichnet, dass** das Färbemittel (F) mindestens ein aminofunktionalisiertes Silikonpolymer (F-1) mit mindestens einer sekundären Aminogruppe enthält.

9. Verfahren nach einem der Ansprüche 1 bis 8, **dadurch gekennzeichnet, dass** das Färbemittel (F) mindestens ein aminofunktionalisiertes Silikonpolymer (F-1) enthält, das mindestens eine Struktureinheit der Formel (Si-Amino) umfasst,

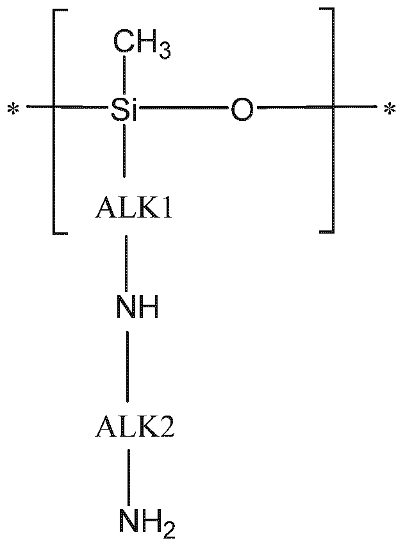

(Si-Amino)

wobei

ALK1 und ALK2 unabhängig voneinander für eine lineare oder verzweigte, zweiwertige $C_1$-$C_{20}$-Alkylengruppe stehen.

10. Verfahren nach einem der Ansprüche 1 bis 9, **dadurch gekennzeichnet, dass** das Färbemittel (F) mindestens ein aminofunktionalisiertes Silikonpolymer (F-1) enthält, das Struktureinheiten der Formel (Si-I) und der Formel (Si-II) umfasst

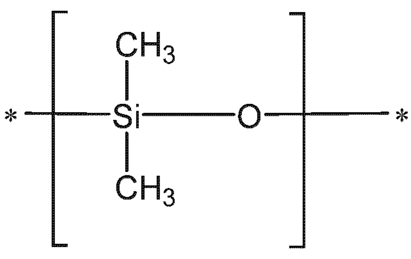

(Si-I)

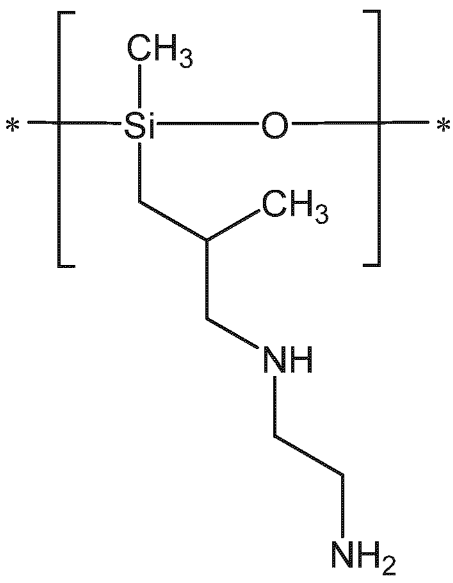

(Si-II).

11. Verfahren nach einem der Ansprüche 1 bis 10, **dadurch gekennzeichnet, dass** das Färbemittel (F) - bezogen auf das Gesamtgewicht des Färbemittels - ein oder mehrere aminofunktionalisierte Silikonpolymere (F-1) in einer Gesamtmenge von 0,1 bis 8,0 Gew.-%, bevorzugt 0,2 bis 5,0 Gew.-%, weiter bevorzugt von 0,3 bis 3,0 Gew.-% und

ganz besonders bevorzugt von 0,4 bis 2,5 Gew.-% enthält.

**12.** Verfahren nach einem der Ansprüche 1 bis 11, **dadurch gekennzeichnet, dass** das Färbemittel (F) mindestens anorganisches Pigment (F-2) enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

**13.** Verfahren nach einem der Ansprüche 1 bis 12, **dadurch gekennzeichnet, dass** das Färbemittel (F) mindestens ein organisches Pigment (F-2) enthält, das bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

**14.** Verfahren nach einem der Ansprüche 1 bis 13, **dadurch gekennzeichnet, dass** das Färbemittel (F) - bezogen auf das Gesamtgewicht des Färbemittels - ein oder mehrere Pigmente (F-2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

## Claims

**1.** A method for dyeing keratinous fibers, in particular human hair, comprising the following steps in the specified order:

(1) applying a pretreatment agent (V) to the keratinous fibers, the pretreatment agent containing at least one anionic surfactant in a water-containing carrier (V-1),
(2) exposing the pretreatment agent applied in step (1) to the keratinous fibers,
(3) rinsing out the pretreatment agent with water,
(4) applying a dye (F) to the keratinous fibers, the dye (F-1) containing at least one amino-functionalized silicone polymer and (F-2) containing at least one pigment,
(5) exposing the dye applied in step (4) to the keratinous fibers, and
(6) rinsing out the dye with water,
**characterized in that** there is a maximum period of 3 hours between steps (3) and (4).

**2.** The method according to claim 1, **characterized in that** the pretreatment agent (V) (V-1) contains at least one anionic surfactant of the formula (T-1),

$$R_1 - \left( O - CH_2 - CH_2 \right)_X - O - SO_3M \qquad \text{(T-1),}$$

where

$R^1$ represents a linear or branched, saturated or unsaturated $C_8$-$C_{30}$ alkyl group,
X represents an integer from 0 to 50, and
M represents a hydrogen atom, ammonium $(NH_4)+$ or an equivalent of a monovalent or polyvalent cation.

**3.** The method according to claim 2, **characterized in that** the pretreatment agent (V) contains at least one anionic surfactant (V-1) of the formula (T-1), where

R represents a linear, saturated or unsaturated $C_{12}$-$C_{18}$ alkyl group,

x represents an integer from 0 to 5, preferably an integer from 1 to 5,
M represents a hydrogen atom or an alkali metal cation, preferably a sodium cation or a potassium cation.

4. The method according to one of claims 1 to 3, **characterized in that** the pretreatment agent (V) contains, based on the total weight of the pretreatment agent, one or more anionic surfactants (V-1) in a total amount of from 2.0 to 18.0 wt.%, preferably from 4.0 to 16.0 wt.%, more preferably from 6.0 to 14.0 wt. %, and very particularly preferably from 8.0 to 12.0 wt.%.

5. The method according to one of claims 1 to 4, **characterized in that** the pretreatment agent (V) (V-2) contains at least one zwitterionic and/or amphoteric surfactant.

6. The method according to claim 5, **characterized in that** the pretreatment agent (V)

(V-2) contains at least one zwitterionic surfactant which is selected from the surfactants of the formula (T-2), (T-3), (T-4) and/or (T-5),

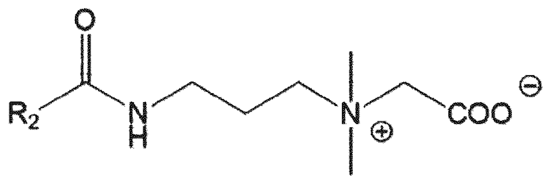

(T-2)

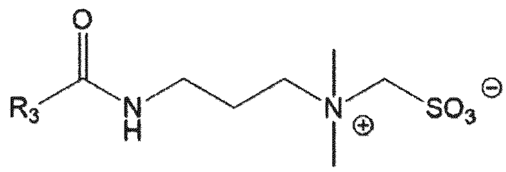

(T-3)

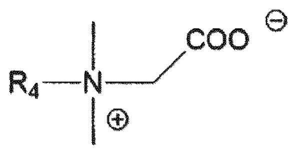

(T-4)

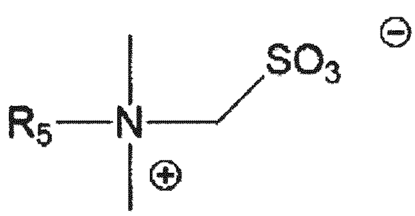

(T-5)

where
R2, R3, R4, R5 independently of one another represent a linear or branched, saturated or unsaturated $C_8$-$C_{30}$ alkyl group, preferably a linear, saturated or unsaturated $C_{12}$-$C_{18}$ alkyl group.

7. The method according to one of claims 1 to 6, **characterized in that** the pretreatment agent (V) contains, based on the total weight of the pretreatment agent, one or more zwitterionic and/or amphoteric surfactants (V-2) in a total amount of from 0.5 to 8.5 wt.%, preferably from 1.0 to 7.5 wt.%, more preferably from 1.5 to 6.5 wt.%, and very

particularly preferably from 2.0 to 4.5 wt.%.

8. The method according to one of claims 1 to 7, **characterized in that** the dye (F) contains at least one amino-functionalized silicone polymer (F-1) having at least one secondary amino group.

9. The method according to one of claims 1 to 8, **characterized in that** the dye (F) contains at least one amino-functionalized silicone polymer (F-1), which comprises at least one structural unit of the formula (Si-amino),

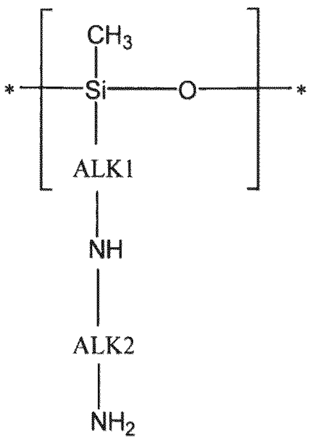

(Si-Amino)

where
ALK1 and ALK2 represent, independently of one another, a linear or branched, bivalent $C_1$-$C_{20}$ alkylene group.

10. The method according to one of claims 1 to 9, **characterized in that** the dye (F) contains at least one amino-functionalized silicone polymer (F-1), which comprises structural units of the formula (Si-I) and of the formula (Si-II).

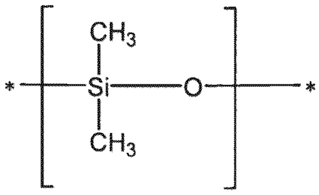

(Si-I)

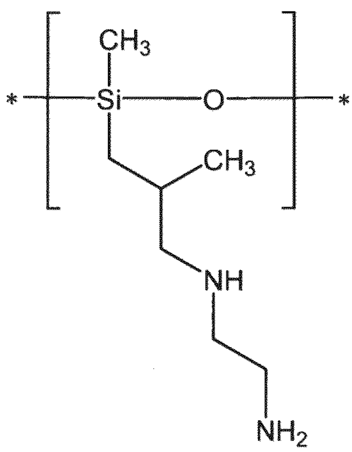

(Si-II).

11. The method according to one of claims 1 to 10, **characterized in that** the dye (F) contains, based on the total weight of the dye, one or more amino-functionalized silicone polymers (F-1) in a total amount of from 0.1 to 8.0 wt.%, preferably from 0.2 to 5.0 wt.%, more preferably from 0.3 to 3.0 wt.%, and very particularly preferably from 0.4 to 2.5 wt.%.

12. The method according to one of claims 1 to 11, **characterized in that** the dye (F) contains at least one inorganic pigment (F-2) which is preferably selected from the group of colored metal oxides, metal hydroxides, metal oxide hydrates, silicates, metal sulfides, complex metal cyanides, metal sulfates, bronze pigments and/or from mica-based colored pigments which are coated with at least one metal oxide and/or metal oxychloride.

13. The method according to one of claims 1 to 12, **characterized in that** the dye (F) contains at least one organic pigment (F-2), which is preferably selected from the group of carmine, quinacridone, phthalocyanine, sorghum, blue

pigments having the Color Index Numbers CI 42090, CI 69800, CI 69825, CI 73000, CI 74100, CI 74160, yellow pigments having the Color Index Numbers CI 11680, CI 11710, CI 15985, CI 19140, CI 20040, CI 21100, CI 21108, CI 47000, CI 47005, green pigments having the Color Index Numbers CI 61565, CI 61570, CI 74260, orange pigments having the Color Index Numbers CI 11725, CI 15510, CI 45370, CI 71105, red pigments having the Color Index Numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

**14.** The method according to one of claims 1 to 13, **characterized in that** the dye (F) contains, based on the total weight of the dye, one or more pigments (F-2) in a total amount of from 0.01 to 10.0 wt.%, preferably 0.1 to 5.0 wt.%, more preferably from 0.2 to 2.5 wt.%, and very particularly preferably from 0.25 to 1.5 wt.%.

**Revendications**

**1.** Procédé permettant de colorer des fibres kératiniques, en particulier des cheveux humains, comprenant les étapes suivantes, dans l'ordre indiqué :

(1) application d'un agent de prétraitement (V) sur les fibres kératiniques, dans lequel l'agent de prétraitement contient (V-1) au moins un tensioactif anionique dans un support aqueux,
(2) action de l'agent de prétraitement appliqué à l'étape (1) sur les fibres kératiniques,
(3) rinçage de l'agent de prétraitement avec de l'eau,
(4) application d'un agent de coloration (F) sur les fibres kératiniques, dans lequel l'agent de coloration contient (F-1) au moins un polymère de silicone aminofonctionnalisé et (F-2) au moins un pigment,
(5) action de l'agent de coloration appliqué à l'étape (4) sur les fibres kératiniques, et
(6) rinçage de l'agent de coloration avec de l'eau,
**caractérisé en ce qu'**une période de 3 heures au maximum s'écoule entre les étapes (3) et (4).

**2.** Procédé selon la revendication 1, **caractérisé en ce que** l'agent de prétraitement (V) contient (V-1) au moins un tensioactif anionique de formule (T-1),

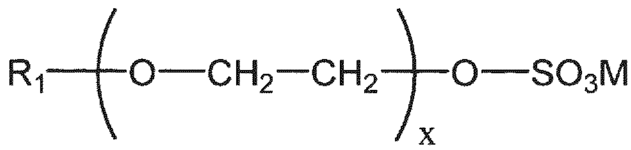

(T-1),

où

$R_1$ représente un groupe alkyle en $C_8$-$C_{30}$ linéaire ou ramifié, saturé ou insaturé,
x représente un nombre entier allant de 0 à 50, et
M représente un atome d'hydrogène, de l'ammonium $(NH_4)_+$ ou un équivalent d'un cation monovalent ou polyvalent.

**3.** Procédé selon la revendication 2, **caractérisé en ce que** l'agent de prétraitement (V) contient au moins un tensioactif anionique (V-1) de formule (T-1), où

R représente un groupe alkyle en $C_{12}$-$C_{18}$ linéaire, saturé ou insaturé,
x représente un nombre entier allant de 0 à 5, de préférence un nombre entier allant de 1 à 5,
M représente un atome d'hydrogène ou un cation de métal alcalin, de préférence un cation de sodium ou un cation de potassium.

**4.** Procédé selon l'une des revendications 1 à 3, **caractérisé en ce que** l'agent de prétraitement (V) contient, par rapport au poids total de l'agent de prétraitement, un ou plusieurs tensioactifs anioniques (V-1) en une quantité totale allant de 2,0 à 18,0 % en poids, de préférence de 4,0 à 16,0 % en poids, plus préférablement de 6,0 à 14,0 % en poids et de manière particulièrement préférée de 8,0 à 12,0 % en poids.

5. Procédé selon l'une des revendications 1 à 4, **caractérisé en ce que** l'agent de prétraitement (V) contient (V-2) au moins un tensioactif zwitterionique et/ou amphotère.

6. Procédé selon la revendication 5, **caractérisé en ce que** l'agent de prétraitement (V) contient

(V-2) au moins un tensioactif zwitterionique qui est choisi parmi les tensioactifs de formule (T-2), (T-3), (T-4) et/ou (T-5),

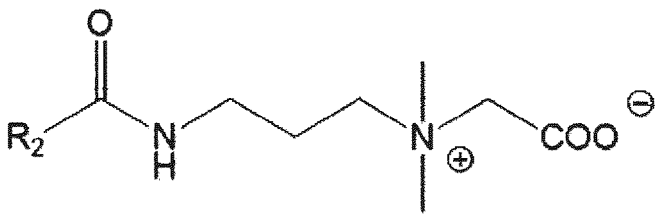

(T-2)

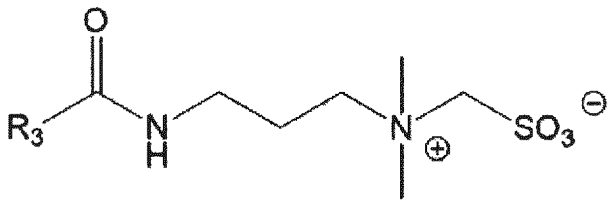

(T-3)

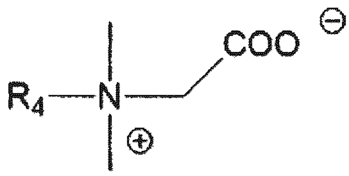

(T-4)

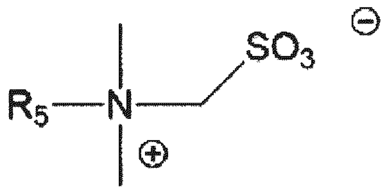

(T-5)

où
R2, R3, R4, R5 représentent, indépendamment les uns des autres, un groupe alkyle en $C_8$-$C_{30}$ linéaire ou ramifié, saturé ou insaturé, de préférence un groupe alkyle en $C_{12}$-$C_{18}$ linéaire, saturé ou insaturé.

7. Procédé selon l'une des revendications 1 à 6, **caractérisé en ce que** l'agent de prétraitement (V) contient, par rapport au poids total de l'agent de prétraitement, un ou plusieurs tensioactifs (V-2) zwitterioniques et/ou amphotères en une quantité totale allant de 0,5 à 8,5 % en poids, de préférence de 1,0 à 7,5 % en poids, plus préférablement de 1,5 à 6,5 % en poids et de manière particulièrement préférée de 2,0 à 4,5 % en poids.

8. Procédé selon l'une des revendications 1 à 7, **caractérisé en ce que** l'agent de coloration (F) contient au moins un polymère de silicone aminofonctionnalisé (F-1) comportant au moins un groupe amine secondaire.

9. Procédé selon l'une des revendications 1 à 8, **caractérisé en ce que** l'agent de coloration (F) contient au moins

un polymère de silicone aminofonctionnalisé (F-1) qui comprend au moins un motif structural de formule (Si-Amino),

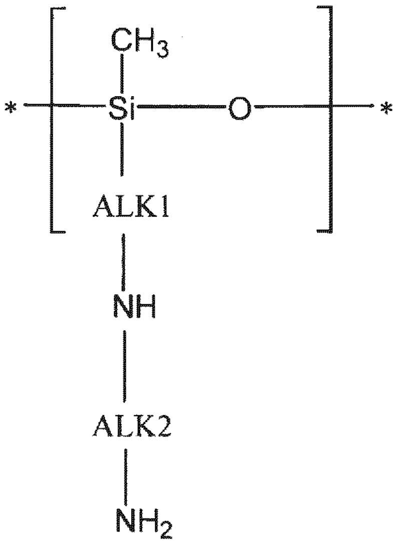

(Si-Amino)

où
ALK1 et ALK2 représentent, indépendamment l'un de l'autre, un groupe alkylène en $C_1$-$C_{20}$ divalent, linéaire ou ramifié.

**10.** Procédé selon l'une des revendications 1 à 9, **caractérisé en ce que** l'agent de coloration (F) contient au moins un polymère de silicone aminofonctionnalisé (F-1) qui comprend des motifs structuraux de formule (Si-I) et de formule (Si-II)

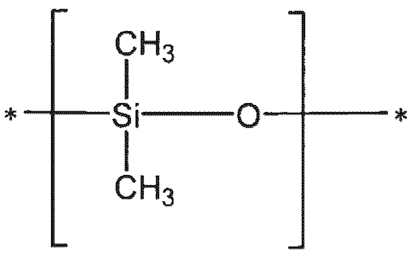

(Si-I)

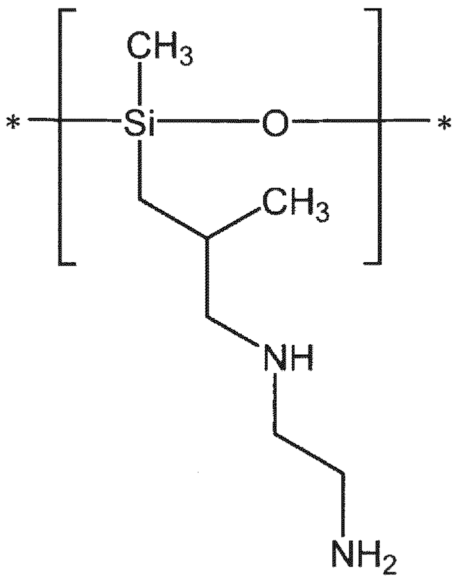

(Si-II).

**11.** Procédé selon l'une des revendications 1 à 10, **caractérisé en ce que** l'agent de coloration (F) contient, par rapport au poids total de l'agent de coloration, un ou plusieurs polymères de silicone aminofonctionnalisés (F-1) en une quantité totale allant de 0,1 à 8,0 % en poids, de préférence de 0,2 à 5,0 % en poids, plus préférablement de 0,3 à 3,0 % en poids et de manière particulièrement préférée de 0,4 à 2,5 % en poids.

**12.** Procédé selon l'une des revendications 1 à 11, **caractérisé en ce que** l'agent de coloration (F) contient au moins un pigment (F-2) inorganique qui est de préférence choisi dans le groupe constitué d'oxydes métalliques colorés, hydroxydes métalliques, oxydes métalliques hydratés, silicates, sulfures métalliques, cyanures métalliques complexes, sulfates métalliques, pigments de bronze et/ou de pigments colorés à base de mica qui sont recouverts

d'au moins un oxyde métallique et/ou d'un oxychlorure métallique.

**13.** Procédé selon l'une des revendications 1 à 12, **caractérisé en ce que** l'agent de coloration (F) contient au moins un pigment (F-2) organique qui est de préférence choisi dans le groupe constitué de carmin, quinacridone, phtalocyanine, sorgho, pigments bleus comportant les numéros d'indice de couleur CI 42090, CI 69800, CI 69825, CI 73000, CI 74100, CI 74160, pigments jaunes comportant les numéros d'indice de couleur CI 11680, CI 11710, CI 15985, CI 19140, CI 20040, CI 21100, CI 21108, CI 47000, CI 47005, pigments verts comportant les numéros d'indice de couleur CI 61565, CI 61570, CI 74260, pigments orange comportant les numéros d'indice de couleur CI 11725, CI 15510, CI 45370, CI 71105, pigments rouges comportant les numéros d'indice de couleur CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

**14.** Procédé selon l'une des revendications 1 à 13, **caractérisé en ce que** l'agent de coloration (F) contient, par rapport au poids total de l'agent de coloration, un ou plusieurs pigments (F-2) en une quantité totale allant de 0,01 à 10,0 % en poids, de préférence de 0,1 à 5,0 % en poids, plus préférablement de 0,2 à 2,5 % en poids et de manière particulièrement préférée de 0,25 à 1,5 % en poids.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- US 2016235655 A1


### In der Beschreibung aufgeführte Nicht-Patentliteratur


- *CHEMICAL ABSTRACTS,* 68891-38-3
- *CHEMICAL ABSTRACTS,* 66455-29-6
- *CHEMICAL ABSTRACTS,* 106842-44-8
- *CHEMICAL ABSTRACTS,* 7558-80-7
- *CHEMICAL ABSTRACTS,* 10049-21-5
- *CHEMICAL ABSTRACTS,* 13472-35-0
- *CHEMICAL ABSTRACTS,* 7758-11-4
- *CHEMICAL ABSTRACTS,* 16788-57-1
- *CHEMICAL ABSTRACTS,* 7558-79-4
- *CHEMICAL ABSTRACTS,* 10028-24-7
- *CHEMICAL ABSTRACTS,* 7782-85-6
- *CHEMICAL ABSTRACTS,* 10039-32-4
- *CHEMICAL ABSTRACTS,* 151-21-3